(19) Europäisches Patentamt
European Patent Office
Office européen des brevets

(11) EP 4 656 729 A1

(12) EUROPEAN PATENT APPLICATION

(43) Date of publication:
03.12.2025 Bulletin 2025/49

(21) Application number: 24179299.3

(22) Date of filing: 31.05.2024

(51) International Patent Classification (IPC):
C12P 5/02 (2006.01)          C12P 19/02 (2006.01)
C12P 19/14 (2006.01)

(52) Cooperative Patent Classification (CPC):
C12P 5/023; C12P 19/02; C12P 19/14

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Renescience A/S
7000 Fredericia (DK)

(72) Inventor: Hansen, Anna Granly
7000 Fredericia (DK)

(74) Representative: IP-Safe ApS
Banegårdspladsen 1
1570 Copenhagen V (DK)

(54) METHOD OF PROCESSING CELLULOSIC MATERIAL

(57) The present invention relates to a method for processing a cellulosic material, use of cellulosic material from waste for producing a bioliquid suitable as a biogas substrate as well as a biogas substrate obtained from a cellulosic material contaminated with biodegradable organic matter. In particular, the present invention relates to a method for processing a cellulosic material and producing a bioliquid, which is suitable as a methane substrate from contaminated cellulosic material of waste, such as municipal solid waste (MSW).

Fig. 1

EP 4 656 729 A1

**Description**

**Technical field of the invention**

The present invention relates to a method for processing a cellulosic material, use of cellulosic material for producing a bioliquid suitable as a biogas substrate as well as a biogas substrate obtained from a cellulosic material, which may be contaminated with biodegradable organic matter and non-biodegradable matter. In particular, the present invention relates to a method for processing a cellulosic material and producing a bioliquid, which is suitable as a methane substrate, from cellulosic material.

**Background of the invention**

There is, in many countries throughout the world, an increased recycling and reuse of various waste fractions, including paper and cardboard, which may partly be due to legislation aiming at increasing recycling and reuse of waste material/fractions, such as paper and cardboard. However, the source sorting and collection of paper and cardboard only applies to paper and cardboard materials, which are not soiled or contaminated with organic or inert material. Consequently, the contaminated paper and cardboard fractions are mostly incinerated or landfilled, which is an ineffective way of utilizing the energy potential in these fractions. In addition, to the source sorted contaminated paper and cardboard, cellulosic material form a significant proportion of the organic fraction of MSW, from which, it is advantageous to effectively utilize the resources and the stored energy within the organic material instead of disposing or incineration of the material, which less effectively utilizes the stored energy. Also, leftover fractions from e.g., pulping of paper and paper mills, such as paper sludge wastes, which contain fibres which are too short for paper production, holds a biogas potential, are currently not harvested or utilized.

Pulp and paper sludge is the main organic residual generated from the wastewater treatments of the pulp and paper industry. The annual world production of paper and paperboard is estimated to 400 million tons in 2015 and is predicted to reach up to 550 million tons by 2050, which could increase the Pulp and paper sludge production by 48-86% compared with the actual rates (Mabee and Roy, 2003). Pulp and paper sludge management and disposal involve economic, environmental, and social costs that will likely increase in the future. Some jurisdictions actually tend to reduce or ban landfilling of organic residuals by improving the efficiency of resource use and recycling.

Considerable interest has arisen in development of efficient and environmentally friendly methods of processing various waste fractions, to maximize recovery of their inherent energy potential in such fractions and, also, to recover recyclable materials. When running single streams of waste fractions, such as cardboard and paper, the first step is to separate the fraction from unsorted waste or to collect the fraction from site sorted sources. The main challenges with site sorting and with waste from restaurants and other industries are that the waste producer may find it difficult and tedious to sort in different waste bins and often the sorted waste is not sorted completely. One specific challenge is when the waste fractions are mixed or polluted with non-cellulosic material. Applicant have previously described, e.g., in WO2013/185778, WO2013/185777 and WO2014198274, the utilisation of organic material from unsorted waste to generate biogas which may be utilized e.g., to create green electricity for households.

In addition to the private households, many industries such as restaurants produce huge amounts of polluted/contaminated paper and cardboard, such as pizza trays, food paper wrappings, cups for hot and cold beverages etc. which are currently not recycled or converted into green energy, since paper and cardboard waste treating facilities, such as paper mills and similar pulping plant for cardboard, are not able to process polluted/contaminated cellulose material, such as paper and cardboard.

Currently used methods for treatment and subsequent disposal of cellulosic material from unsorted waste, such as household, agricultural or municipal waste, include among others, Anaerobic Digestion (AD), incineration, landfill, mechanical and biological treatment, where the method of choice often depends on e.g., the content of organic material compared to the content of non-organic material. However, none of these methods can effectively utilize the energy potential stored in the the contaminated cellulosic fraction of this waste fraction.

Anaerobic Digestion (AD) are widely used to produce methane from biomass. AD-processing of cellulosic material with organic material includes solid-state AD processing of a mixture of fibers (cardboard, boxboard, newsprint, and fine paper) with varying amounts of food waste (Guilford., et.al. Environ. Sci. Technol. 2019, 53) and Co-digestion of food waste with other C-rich materials, such as paper and cardboard (Shahbaz et.al. Appl. Sci. 2, 1436, 2020). However, AD processing of paper and cardboard are challenging due to the lignin content of the lignocellulosic material, leading to ineffective solubilisation and utilization of the methane potential, Naroznova., et.al. Waste Management, Volume 50, 2016. Thus, anaerobic digestion of lignocellulosic material usually includes chemical or mechanical pretreatment to solubilize the lignin content. An example of biological pretreatment of paper and cardboard with microbial consortium prior to AD-processing is described in Yuan et.al. Bioresource Technology, Volume 118, August 2012). Pretreatment as described above, may be applied but are expensive and time consuming. Additionally, the AD-processing may also be affected by the processes used to make the paper (Gonzalez-Estrella, et.al. Rev Environ Sci Biotechnol 16, 2017).

Where the unsorted cellulosic material comprised in household waste can be processed by the method of Applicant i.e. as described in WO2013/185778, WO2013/185777 and WO2014198274, then already partly sorted or source generated cellulosic material comprising high amount of cellulose such as paper and cardboard are currently not treated by processes utilizing the energy potential, e.g. to generate biogas.

The present inventors have found that the cellulosic material from waste e.g., industries such as restaurant, household waste either unsorted or sorted e.g., into the paper and/or cardboard bin, can be processed according to a method based on enzymatic hydrolysis and/or microbial fermentation. Applying a method of enzymatic hydrolysis and/or microbial fermentation to the cellulosic fraction effectively utilizes the high energy potential in the fraction. This is surprising, as this fraction up to now has been considered unsuitable for effective conversion to energy. In particular, this fraction is unsuitable for processing in e.g., paper mills and other pulping facilities. Processing e.g., contaminated cellulosic material according to the method of the invention provides improved energy recovery from a waste fraction, which until now have not been sufficiently utilized in current treatment methods.

The present invention provides a method for efficient energy recovery from the cellulosic material and in particular contaminated cellulosic material. The invention further provides a bioliquid obtained by treating the contaminated cellulosic material, using the method of the present invention, which gives rise to a significant high methane potential and in some embodiments even an improved methane potential compared to the methane potential obtained using unsorted waste, such as municipal solid waste (MSW). Thus, the inventors have surprisingly found that the contaminated cellulosic material can be effectively processed, and the energy potential released and in addition, that the cellulosic materials have significant biogas potential when processed according to the method of the present invention. Further, the bioliquid from the material may even have a higher biogas e.g., methane potential, biofuel or chemical production potential, than bioliquid from unsorted waste, such as MSW.

This is advantageous, since the cellulosic material, in particular, if contaminated, as mentioned, in general is considered a problematic waste fraction, which cannot be recycled or reused when processed in current pulping methods. By sorting or isolating, the cellulosic material and subsequently processing the cellulosic material, a potent bioliquid with high energy potential can be obtained and processed to provide green energy products, such as biogas.

Thus, compared to current treatment methods used in the industry, the method of the invention provides an improvement of energy recovery from cellulosic material, e.g., originating from site sourced waste, industry waste or household waste. The method of processing cellulosic material using enzymatic hydrolysis and/or microbial fermentation provides a bioliquid with a surprisingly high methane potential.

## Summary of the invention

Thus, one aspect of the invention relates to a method for processing cellulosic material, wherein the method comprises the steps of:

(i) adding a stream of cellulosic material, such as paper, cardboard, pizza trays, carton, newspapers, magazines, office paper, diapers, pulp or paper sludge, to a bioreactor and optionally add liquid, wherein the cellulosic material has a dry matter content of cellulose of at least 20% by dry weight;

(ii) further add an enzyme comprising one or more cellulase enzymes selected from the group comprising an endoglucanase, a cellobiohydrolase, and a beta-glucosidase; followed by

(iii) enzymatic hydrolysis of the cellulosic material within the bioreactor at a temperature in the range of 35-75 °C and pH 4-7 for 2-48 hours resulting in liquefaction of the biodegradable organic matter and the cellulosic material to produce a bioliquid; optionally followed by

(iv) separation of liquid and solid; optionally followed by

(v) anaerobic fermentation of the bioliquid into a biogas.

Another aspect of the present invention relates to the Use of a cellulosic material for producing a bioliquid suitable as a biogas substrate comprising the steps of:

(i) adding a stream of cellulosic material, such as paper, cardboard, pizza trays, carton, newspapers, magazines, office paper, diapers, pulp or paper sludge, to a bioreactor and optionally add liquid, wherein the cellulosic material has a dry matter content of cellulose of at least 20% by dry weight;

(ii) further add an enzyme comprising one or more cellulase enzymes selected from the group comprising an endoglucanase, a cellobiohydrolase, and a beta-glucosidase and one or more enzymes selected from the list comprising a protease, a lipase, a beta-glucanase, a pectate lyase, a hemicellulase, a mannanase, and an amylase; followed by

(iii) enzymatic hydrolysis of the cellulosic material within the bioreactor at a temperature in the range of 35-75 °C and pH 4-7 for 2-48 hours resulting in liquefaction of the biodegradable organic matter and the cellulosic material to produce a bioliquid; optionally followed by

(iv) separation of liquid and solid; optionally followed by

(v) anaerobic fermentation of the bioliquid into a biogas.

Yet another aspect of the present invention relates to a biogas substrate obtained from a cellulosic material obtained by the process of:

(i) adding a stream of cellulosic material, such as paper, cardboard, pizza trays, carton, newspapers, magazines, office paper, diapers, pulp or paper sludge, to a bioreactor and optionally add liquid, wherein the cellulosic material has a dry matter content of cellulose of at least 20% by dry weight;

(ii) further add an enzyme comprising one or more cellulase enzymes selected from the group comprising an endoglucanase, a cellobiohydrolase, and a beta-glucosidase and one or more enzymes selected from the list comprising a protease, a lipase, a beta-glucanase, a pectate lyase, a hemicellulase, a mannanase, and an amylase; followed by

(iii) enzymatic hydrolysis of the cellulosic material within the bioreactor at a temperature in the range of 35-75 °C and pH 4-7 for 2-48 hours resulting in liquefaction of the biodegradable organic matter and the cellulosic material to produce a bioliquid; optionally followed by

(iv) separation of liquid and solid; optionally followed by

(v) anaerobic fermentation of the bioliquid into a biogas.

**Brief description of the figures**

Figure 1 shows photos of the cellulosic fractions used in the examples. The effect of fractions sizes (dimensions) was assessed as follows: the cellulosic fractions were cut into larger (also referred to as "cut") and smaller pieces (also referred to as "shred"). The large pieces were at least 1 cm wide and 10 cm long **(Fejl! Henvisningskilde ikke fundet.** a, d, e, g) while the small pieces were at max 0.5 cm wide and 7-8 cm long **(Fejl! Henvisningskilde ikke fundet.** b, c, f, h).

Figure 2 shows the composition of the cellulosic fractions from the strong acid hydrolysis.

Figure 3 shows the average composition of selected fractions from the literature.

Figure 4 shows the effect of pre-soaking the fractions in warm water. When pre-soaked in warm water for 3 hours prior to adding enzymes, all the tested fractions seem to release less COD in comparison to adding enzymes directly to the fractions. Because the pre-soaking experiments were not replicated it is difficult to conclude whether this is an effect of pre-soaking alone.

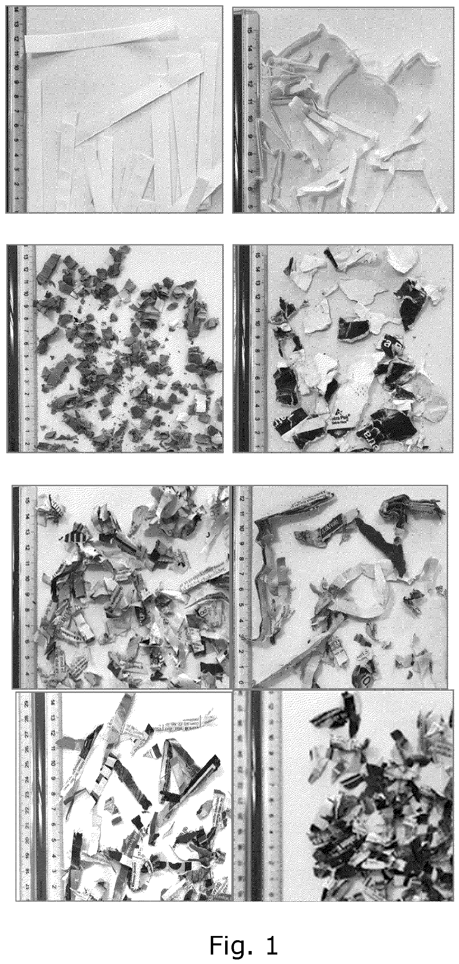

Figure 1 shows the effect of fraction size on dissolved COD. When cut in bigger pieces all the tested fractions seem to release less COD in comparison to when being shredded. Because the experiments were not replicated it is difficult to conclude whether this is an effect of the fraction size alone.

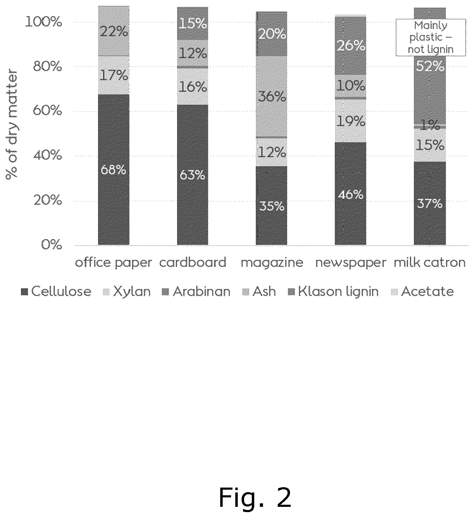

Figure 2 shows a comparison of total and dissolved COD (with the contribution of enzymes subtracted) at different enzyme dosages. Both total and dissolved COD did not reduce proportionally to the reduction of enzyme dosage. In addition, total COD reduced very little at low enzyme dosages suggesting that even though enzymes did not manage to fully dissolve the fractions still managed to destroy their structure to small enough pieces to be detectable by the total COD measurements.

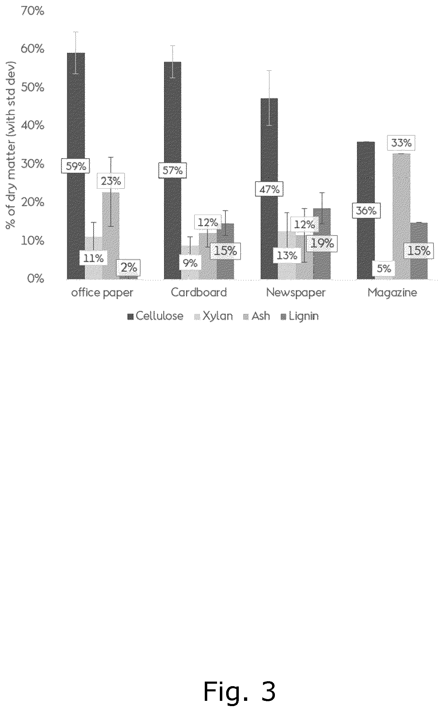

Figure 3 shows actual COD values measured for all the fractions at different enzyme loadings. While dissolved COD differed a lot at different conditions, total COD was relative similar which suggests that enzymes played two roles: dissolved the organic material in the fractions and destroy the structure of the fractions to give small enough particles detectable by the total COD measurements.

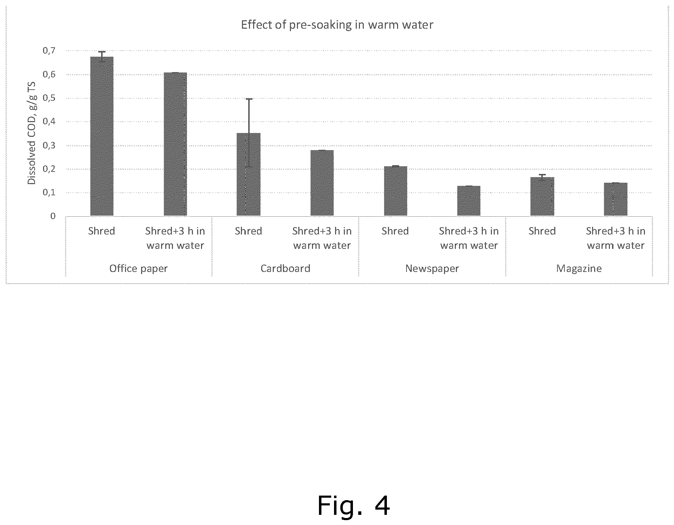

Figure 4 shows dissolved COD released from the cellulosic fractions mixed with model waste. When hydrolysed with model waste, the tested fractions yield a higher dissolved COD in comparison to model waste alone.

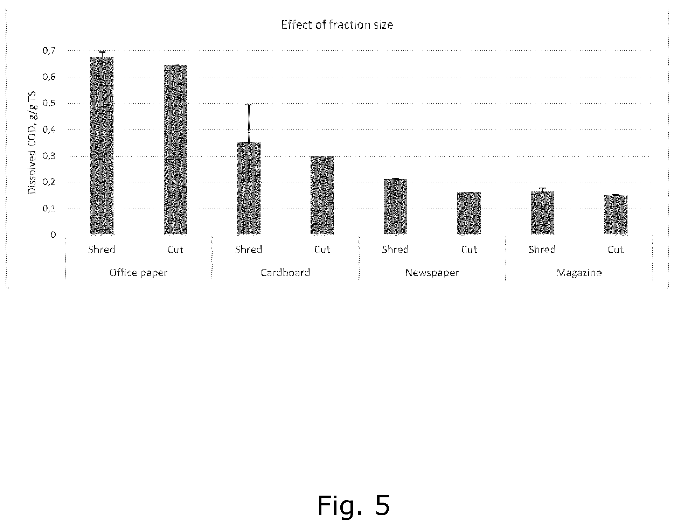

Figure 5 shows dissolved COD released from the fractions enzymatically hydrolysed in tap water or in reject water acidified with acetic acid. Dissolved COD is comparable for these two-reaction media, which suggests that the acidification of reject water did not affect the performance of enzymes.

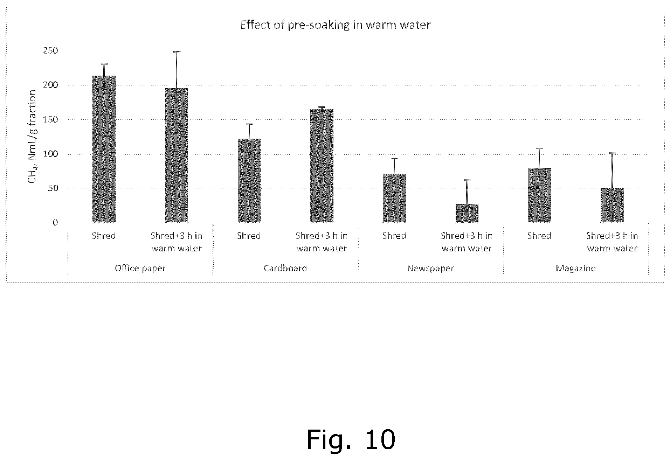

Figure 10 shows the effect of pre-soaking of the fractions in warm water on the methane production. The results show that pre-soaking had almost no effect on the methane production from a fraction.

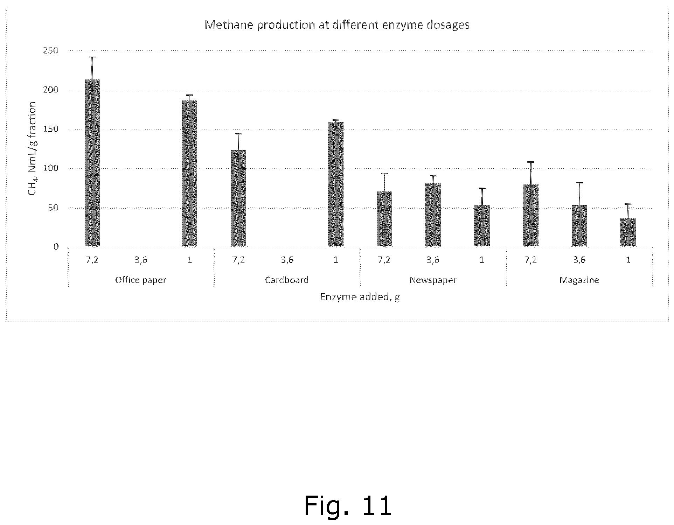

Figure 11 illustrates methane production at different enzyme dosages.

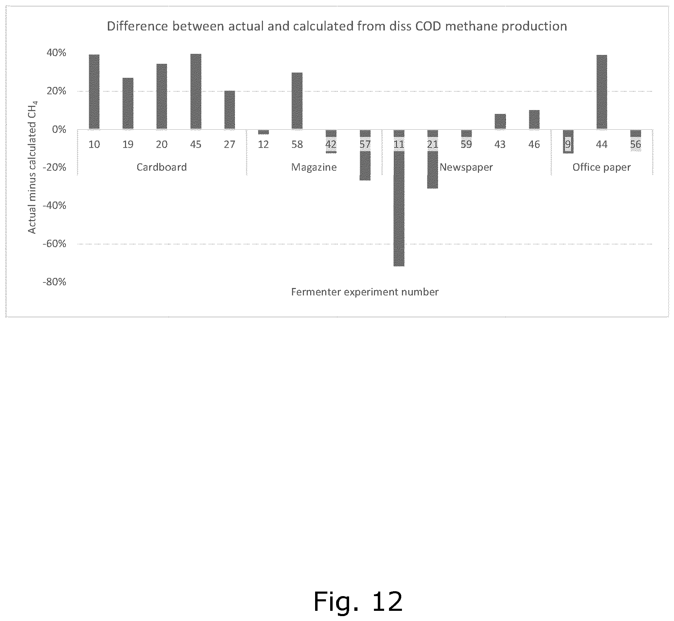

Figure 12 shows difference between actual and calculated from dissolved COD methane production. Suspended COD has a large contribution on the experiments with a large positive difference between the actual and calculated $CH_4$ production while the experiments with a difference close to zero mainly produced biogas from dissolved COD.

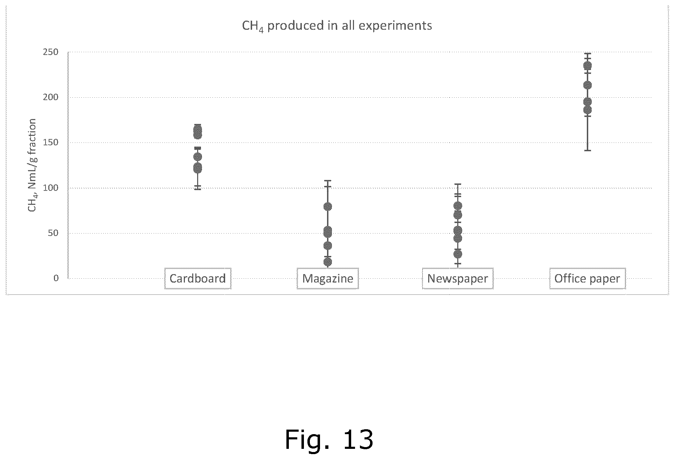

Figure 13 illustrates methane produced in all AMPTS experiments for every fraction (contribution of enzymes and acetic acid was subtracted). The methane production was specific for each fraction.

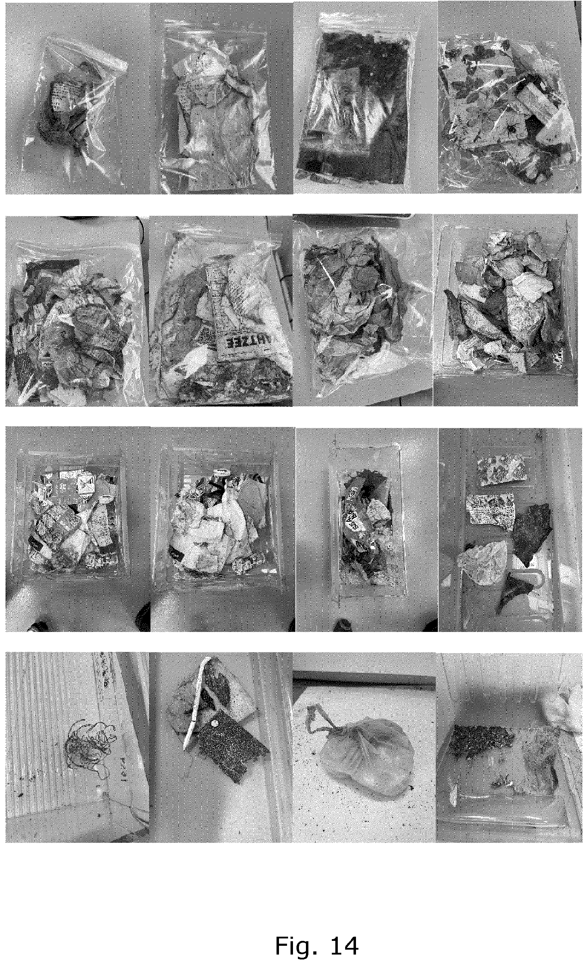

Figure 14 shows pictures of the composition of the contaminated cellulosic material from MSW waste sorted into the following fractions (from left to right):

Newspaper, receipts, fotos/postcards, Birthday/Christmas/visiting cards, magazines, office paper, napkins/paper handkerchiefs, cardboard, juice cartons, window envelopes, soft plastic, hard plastic, metal, wood, plastic bag with used tampons, and soil/hair/feather.

The present invention will now be described in more detail in the following.

## Detailed description of the invention

The present invention provides a method for processing of cellulosic material and utilising the energy potential of this material. The inventors have found that the bioliquid from cellulosic material, in particular contaminated cellulosic material, e.g., originating from waste, e.g., waste from food packages, such as pizza boxes and coffee cups, and other cellulosic material used as food and beverage containers, have significant energy potential. The energy potential of the cellulosic material is utilized by processing cellulosic material with the enzymatic hydrolysis and/or microbial fermentation, which provides a bioliquid with a surprisingly high energy potential, when used as a methane substrate.

Thus, the present inventors have found that cellulosic material and in particular contaminated cellulosic material can be processed effectively according to the method of the invention and provides surprising energy potential e.g., measured as methane potential, when treated according to the method of the present invention.

The bioliquid obtained by treating cellulosic material gives rise to an improved methane potential compared to the methane potential of bioliquid obtained from processing unsorted waste e.g., MSW by the same method.

The cellulosic material fraction may come directly from mixed or unsorted waste e.g., after sorting or the cellulosic material may be derived from a site sorted fraction e.g., specific waste bin, directly from industry such as restaurants collecting contaminated paper and cardboard or the cellulosic material may derive from food and beverage shops, such as coffee shops, where the contaminated cups and packaging are collected or disposed. Thus, cellulosic material suitable for processing according to the method described herein may derive from any of these sources. A bioliquid with high energy potential results from treatment of cellulosic material in an enzymatic hydrolysis and/or microorganism process as described in the present invention, preferably an enzymatic hydrolysis and/or microorganism process, wherein the cellulosic material are paper e.g., magazines, paper boxes, box board, corrugated cardboard, newspapers or paper used for wrapping organic containing material, cardboard used for food or beverage storage including pizza boxes and cups and other food and beverage boxes, wherein the paper or cardboard comprises a large fraction of cellulosic material. The cellulosic material is in this case contaminated with food or other organic material residues from the storage or wrapping.

The high energy potential of the bioliquid from cellulosic material or contaminated cellulosic material renders the method of the invention and resulting bioliquid more beneficial to the environment by increasing the energy output as compared to other methods of treating cellulosic material, which is usually not recycled or reused, and provides at the same time a better overall process economy rendering the final product e.g., bioethanol or biogas such as methane more competitive in terms of lower costs to the end user.

The higher energy potential or density of the bioliquid from cellulosic material or contaminated cellulosic material provides for a higher output of methane per ton bioliquid as compared to other waste or waste fractions when cellulosic material is treated according to the method of the present invention.

In one embodiment the bioliquid result from treatment of cellulosic material in an enzymatic hydrolysis and/or microorganism fermentation, wherein the cellulosic material is derived from paper or cardboard contaminated with organic material. The method of the present invention provides an alternative to treating contaminated cellulose waste, which cannot be pulped to produce new paper or cardboard. Contaminated paper and cardboard are difficult waste fractions as these cannot when pulped be processed into recyclable or reusable products due to the contamination from the organic material. Even if it can be pulped the method of the present invention provides a better alternative to landfill or incineration. It is a new, more sustainable treatment route. The method of the invention solves this problem by providing a method, which effectively utilize the energy potential from the paper and cardboard as such and from the contaminating organic material, by converting the materials into a bioliquid comprising high methane, ethanol, and other potential for green energy.

Thus, one aspect of the invention relates to a method for processing cellulosic material, wherein the method comprises the steps of:

(i) adding a stream of cellulosic material, such as paper, cardboard, pizza trays, carton, newspapers, magazines, office paper, diapers, pulp or paper sludge, to a bioreactor and optionally add liquid, wherein the cellulosic material has a dry matter content of cellulose of at least 20% by dry weight;

(ii) further add an enzyme comprising one or more cellulase enzymes selected from the group comprising an endoglucanase, a cellobiohydrolase, and a beta-glucosidase; followed by

(iii) enzymatic hydrolysis of the cellulosic material within the bioreactor at a temperature in the range of 35-75 °C and pH 4-7 for 2-48 hours resulting in liquefaction of the biodegradable organic matter and the cellulosic material to produce a bioliquid; optionally followed by

(iv) separation of liquid and solid; optionally followed by

(v) anaerobic fermentation of the bioliquid into a biogas.

In one embodiment of the present invention, the contaminated cellulosic material is isolated from waste, such as municipal solid waste.

In one embodiment of the present invention, the cellulosic material is contaminated with biodegradable, and/or inert and/or non-biodegradable material, such as food waste, biogenic material, plastics, sand, metal, non-biodegradable combustible material, material isolated from waste, such as municipal solid waste or sludge, such as pulper or paper sludge, sludge from wastewater treatment plant. The contaminated cellulosic material may be collected from industry, such as food and beverage industry.

In one embodiment of the present invention, the contaminated cellulosic material is paper, such as newspapers and paper used for wrapping organic material.

In one embodiment of the present invention, the contaminated cellulosic material is cardboard, such as pizza trays and other cardboard material used for storing of organic material for a shorter or longer period.

In one embodiment of the present invention, the contaminated cellulosic material is cardboard, such as cups from shops selling coffee and other cold or hot beverages.

In one embodiment of the present invention, the contaminated cellulosic material is obtained from household waste either sorted in separate bin(s) or from mixed or unsorted household waste, e.g., MSW.

In one particular embodiment of the present invention, the contaminated cellulosic material is paper sludge material, i.e., paper material which have already been processed e.g., by pulping.

In another embodiment of the present invention, the contaminated cellulosic material comprises between 20 and 99% by dry weight cellulosic material, preferably between 30 and 80% by weight cellulosic material, more preferably between 40 and 70% by weight cellulosic material of the treated stream on a dry matter basis.

In yet another embodiment of the present invention, the cellulosic material comprises between 30 and 99% by weight cellulose, preferably between 45 and 95% by weight cellulose, more preferably between 50 and 90% by weight cellulose, most preferably between 60 and 90% by weight cellulose on a dry matter basis.

In a further embodiment of the present invention, the cellulosic material comprises between 0 and 40% by weight hemicellulose, such as between 1 and 35% by weight hemicellulose, such as between 5 and 30% by weight hemicellulose, preferably between 1 and 25% by weight hemicellulose, more preferably between 5 and 25% by weight hemicellulose and/or wherein the cellulosic material comprises between 0 and 30% by weight lignin, preferably between 0 and 25% by weight lignin, more preferably between 1 and 25% by weight, most preferably between 5 and 25% by weight lignin on a dry matter basis.

In another embodiment of the present invention, the cellulosic material is preferably mechanically treated such as shredded before the enzymatic hydrolysis, preferably by cutting the cellulosic material into smaller pieces, such as having a screen size between 30 mm to about 400 mm, preferably a screen size between 40 mm to 250 mm, more preferably a screen size between 40 mm to 150 mm, most preferably 100 mm or below. The cellulosic material may be shredded by cutting the cellulosic material into small pieces, such as 5-10 mm wide and 50-100 mm long, preferably 5 mm wide and 80 mm long.

In certain cases, it may be beneficial to pretreat the contaminated cellulosic material to disrupt the cellulosic structures in the material to make it more accessible to enzymatic hydrolysis and liquefaction. Such pretreatment includes heating and/or pressure, and treatment with chemicals such as alkaline solutions.

Thus, in one embodiment of the present invention, the cellulosic material is pretreated for 2-10 hours, preferably 2-5 hours, more preferably 3 hours in water at a temperature of 40-100 degrees C, preferably 50-100 degrees C, more preferably 50-60 degrees C.

In other embodiments contaminated cellulosic material can be pretreated both physically (mechanically) and chemically. Mechanical or physical pretreatment can be coupled with steaming/steam explosion, hydrothermolysis, dilute or mild acid treatment, high temperature, high pressure treatment, irradiation (e.g., microwave irradiation), or combinations thereof. In one aspect, high pressure means pressure in the range of preferably about 100 to about 400 psi, e.g., about 150 to about 250 psi. In another aspect, high temperature means temperature in the range of about 100 to about 300°C, e.g., about 140 to about 200°C. In a preferred aspect, mechanical or physical pretreatment is performed in a batch-process using a steam gun hydrolyzer system that uses high pressure and high temperature as defined above, e.g., a Sunds Hydrolyzer available from Sunds Defibrator AB, Sweden. The physical and chemical pretreatments can be carried out sequentially or simultaneously, as desired.

In some embodiments, it may be beneficial to add waste, preferably organic containing waste, to the liquified cellulosic material before fermentation to promote the fermentation process, because the waste normally comprises suitable microorganisms as well as organic material which will add to the fermentation process. Thus, in one embodiment, waste is added to the liquefied cellulosic material before fermentation.

In one embodiment of the present invention, the cellulosic material from waste has a high methane potential in the range of 20 to 300 L/kg cellulosic material , 25 to 300 L/kg cellulosic material , 40 to 300 L/kg cellulosic material , 50 to 300 L/kg cellulosic material , 60 to 300 L/kg cellulosic material , 70 to 300 L/kg cellulosic material , 80 to 300 L/kg cellulosic material , 90 to 300 L/kg cellulosic material , 20 to 250 L/kg cellulosic material , 50 to 250 L/kg cellulosic material , 70 to 250 L/kg cellulosic material , 90 to 250 L/kg cellulosic material , 100 to 250 L/kg cellulosic material , 125 to 250 L/kg cellulosic material, preferably 150 to 250 L/kg cellulosic material , 175 to 250 L/kg cellulosic material , 200 to 250 L/kg cellulosic material on a dry matter basis.

Thus, in one embodiment, the methane potential of the liquefied cellulosic material is 25-250 L/kg cellulosic material, preferably 50-250 L/kg cellulosic material, more preferably 100-250 L/kg cellulosic material and most preferably 150-250 L/kg cellulosic material on a dry matter basis.

In another embodiment of the present invention, the cellulosic material obtained in step (i) is mixed with liquid in an amount of 500 to 3000 L/ton cellulosic material, 500 - 2000 L/ton cellulosic material, 500 - 1500 L/ton cellulosic material or in the range 500 to 1000 L/ton before concurrent enzymatic hydrolysis and/or microbial fermentation in step (ii).

In one embodiment of the present invention, the cellulosic material comprises a major part of the material originating from cardboard, especially from food and beverage storage boxes, such as pizza boxes, milk cartoon, cups for hot and cold beverage, or similar cardboard sources, wherein the cellulosic material is contaminated with organic food or beverage debris or material.

In one embodiment of the present invention, the non-water or dry matter content of the cellulosic material is between 10 and 80%, between 10 and 70%, between 10 and 60%, between 10 and 55%, between 10 and 50% between 20 and 60%, preferably between 15 and 50%, more preferably between 20 and 45% by weight of cellulosic material.

The liquid used for the liquefaction of the cellulosic material in step (iii) may be any of, water e.g., brown water, rainwater, towns water or water from any other source such as lakes, sea etc. The liquid may further be recycled water or other liquids from the process by which the cellulosic material is liquified. In one embodiment the liquid is recycled bioliquid. In another embodiment of the present invention, microorganism inoculation and/or enzyme hydrolysis of cellulosic material is provided at least partly by recycling wash waters (or liquids used for washing) or liquid process solutions (process water or process liquid). The liquid added in step (ii) is used for liquefaction of cellulosic material as well as to recover residual organic material from non-degradable solids.

Reject water can likewise be used in step (I) as a liquid. When reject water is used, raw reject water is first acidified to around pH 5.7 by using an acid, such as acetic acid, and then added to the fermentation process where pH is further adjusted to around 5.

It may be advantageous to adjust the temperature in the process prior to initiation of enzymatic hydrolysis and/or microbial fermentation in step (iii). In a preferred embodiment of the invention, at least one enzyme used in the hydrolysis of cellulosic material comprises cellulase activity. As is well known in the art, cellulases and other enzymes typically exhibit an optimal activity within the temperature range 30°C - 75°C. The objective of heating may simply be to render the majority of e.g., cellulosic material from waste and a substantial fraction of the cellulosic material from waste in a condition optimal for enzymatic hydrolysis and/or microbial fermentation. To be in a condition optimal for enzymatic hydrolysis and/or microbial fermentation, cellulosic material should ideally have a temperature and water content appropriate for the enzyme activities used for enzymatic hydrolysis. Thus, one embodiment of the present invention, the liquid e.g., water is preheated to a temperature in the range of 30-75 °C, preferably 45-56 °C, more preferably around 50 °C, such as 49-51°C.

It can be advantageous to agitate during heating to achieve evenly heated cellulosic material from waste. Agitation further achieves the introduction of mechanical energy to create shear forces in the cellulosic material. Agitation can comprise free-fall mixing, such as mixing in a reactor, which may have a chamber that rotates along a substantially horizontal axis or in a mixer having a rotary axis lifting the slurry of cellulosic material or in a mixer having horizontal shafts or paddles lifting slurry of cellulosic material. Agitation can comprise one or more of shaking, stirring or conveyance through a transport screw conveyor. The agitation may continue after the slurry of cellulosic material has been heated to the desired temperature.

In a further embodiment of the present invention, if inoculation with microorganisms is performed, the inoculation with microorganisms is made before or concurrently with the addition of enzymatic activities or with the addition of microorganisms that exhibit extra-cellular cellulase activity. In one preferred embodiment the fermentation of the cellulosic material in step (iii) is made by microorganisms already present in the cellulosic material. In one embodiment of the present invention, microbial fermentation of step (ii) is accomplished by inoculation using e.g., lactic acid bacteria, or with bacteria naturally present in the waste. Preferably, microbial fermentation in step (ii) is performed concurrently with the enzymatic hydrolysis.

The enzymes of the enzymatic activities used in the method according to the present invention may comprise cellulase(s) and/or hemicellulase(s) as defined herein, such as one or more of exoglucanases, endoglucanases, endoxylanases, xylosidases, acetyl xylan esterases and beta glucosidases, including any combination thereof. The enzymes are preferably endoglucanase, cellobiohydrolase, and optionally a beta-glucosidase and one or more enzymes selected from the list comprising a protease, a lipase, a beta-glucanase, a pectate lyase, a hemicellulase, a mannanase, and an amylase.

In one embodiment of the present invention, the enzymes are added as recirculated bioliquid, process water, microorganism secreting enzyme, or as an enzyme composition.

In another embodiment of the present invention, the enzymatic hydrolysis is performed using an enzyme composition comprising or further comprising one or more (e.g., several) proteins selected from the group consisting of a cellulase, an AA9 polypeptide, a hemicellulase, an esterase, an expansin, a ligninolytic enzyme, an oxidoreductase, a pectinase, a protease, and a swollenin. In one embodiment, the cellulase is preferably one or more (e.g., several) enzymes selected from the group consisting of an endoglucanase, a cellobiohydrolase, and a beta-glucosidase. In a further embodiment, the hemicellulase is preferably one or more (e.g., several) enzymes selected from the group consisting of an acetylmannan esterase, an acetylxylan esterase, an arabinanase, an arabinofuranosidase, a coumaric acid esterase, a feruloyl esterase, a galactosidase, a glucuronidase, a glucuronoyl esterase, a mannanase, a mannosidase, a xylanase, and a xylosidase. In another embodiment, the oxidoreductase is preferably one or more (e.g., several) enzymes selected from the group consisting of a catalase, a laccase, and a peroxidase.

In yet another embodiment, the enzyme comprises one or more (e.g., several) cellulolytic enzymes. In one embodiment, the enzyme comprises or further comprises one or more (e.g., several) hemicellulolytic enzymes. In another embodiment, the enzyme comprises one or more (e.g., several) cellulolytic enzymes and one or more (e.g., several) hemicellulolytic enzymes. In a further embodiment, the enzyme comprises one or more (e.g., several) enzymes selected from the group of cellulolytic enzymes and hemicellulolytic enzymes. In another embodiment, the enzyme comprises an endoglucanase. In another embodiment, the enzyme comprises a cellobiohydrolase. In another embodiment, the enzyme comprises a beta-glucosidase. In another embodiment, the enzyme comprises an AA9 polypeptide. In another embodiment, the enzyme comprises an endoglucanase and an AA9 polypeptide. In another embodiment, the enzyme comprises a cellobiohydrolase and an AA9 polypeptide. In another embodiment, the enzyme comprises a beta-glucosidase and an AA9 polypeptide. In another embodiment, the enzyme comprises an endoglucanase and a cellobiohydrolase. In another embodiment, the enzyme comprises an endoglucanase and a cellobiohydrolase I, a cellobiohydrolase II, or a combination of a cellobiohydrolase I and a cellobiohydrolase II. In another embodiment, the enzyme comprises an endoglucanase and a beta-glucosidase. In another embodiment, the enzyme comprises a beta-glucosidase and a cellobiohydrolase. In another embodiment, the enzyme comprises a beta-glucosidase and a cellobiohydrolase I, a cellobiohydrolase II, or a combination of a cellobiohydrolase I and a cellobiohydrolase II. In another embodiment, the enzyme comprises an endoglucanase, an AA9 polypeptide, and a cellobiohydrolase. In another embodiment, the enzyme comprises an endoglucanase, an AA9 polypeptide, and a cellobiohydrolase I, a cellobiohydrolase II, or a combination of a cellobiohydrolase I and a cellobiohydrolase II. In another embodiment, the enzyme comprises an endoglucanase, a beta-glucosidase, and an AA9 polypeptide. In another embodiment, the enzyme comprises a beta-glucosidase, an AA9 polypeptide, and a cellobiohydrolase. In another embodiment, the enzyme comprises a beta-glucosidase, an AA9 polypeptide, and a cellobiohydrolase I, a cellobiohydrolase II, or a combination of a cellobiohydrolase I and a cellobiohydrolase II. In another embodiment, the enzyme comprises an endoglucanase, a beta-glucosidase, and a cellobiohydrolase. In another embodiment, the enzyme comprises an endoglucanase, a beta-glucosidase, and a cellobiohydrolase I, a cellobiohydrolase II, or a combination of a cellobiohydrolase I and a cellobiohydrolase II. In another embodiment, the enzyme comprises an endoglucanase, a cellobiohydrolase, a beta-glucosidase, and an AA9 polypeptide. In another embodiment, the enzyme comprises an endoglucanase, a beta-glucosidase, an AA9 polypeptide, and a cellobiohydrolase I, a cellobiohydrolase II, or a combination of a cellobiohydrolase I and a cellobiohydrolase II.

In one embodiment of the present invention, the cellobiohydrolase is one or more enzymes selected from the group comprising a cellobiohydrolase I, a cellobiohydrolase II, and a beta-glucosidase.

In another embodiment of the present invention, the enzyme further comprises one or more enzymes selected from an AA9 polypeptide, a cellulose inducible protein (CIP), an esterase, an expansin, a ligninolytic enzyme, an oxidoreductase, a pectinase, and a swollenin.

In yet another embodiment of the present invention, the hemicellulase is one or more enzymes selected from a xylanase, an acetylxylan esterase, a feruloyl esterase, an arabinofuranosidase, a xylosidase, and a glucuronidase.

The enzymatic activities of the enzymes or cellulolytic enzyme may also be added in an amount of 0.005-5%, such as 0.01-2%, such as in an amount of 0.01-1.5%, such as in an amount of 0.1-1.0%, preferably 0.1-0.9%, more preferably 0.125-0.9% by weight of the cellulosic material.

The enzyme, such as cellulase activity may be added to step (ii) (a) by inoculation with a selected microorganism that exhibits extra-cellular enzyme such as cellulase activity and/or (b) as an isolated enzyme e.g., cellulase preparation.

In an additional embodiment of the present invention, the microbial fermentation if this is accomplished by inoculation use one or more species of lactic acid bacteria.

In one embodiment of the present invention, the enzymatic hydrolysis is performed at a temperature in the range of 30-75 degrees C, preferably 40-60 degrees C, more preferably 45-55 degrees C, most preferably 50 degrees C.

In another embodiment of the present invention, the fermentation is conducted at a temperature in the range of 30-75 degrees C, preferably 40-60 degrees C, more preferably 45-55 degrees C, most preferably 50 degrees C.

In one embodiment of the present invention, the enzymatic hydrolysis is performed for a period of wherein the enzymatic hydrolysis is performed for a period of 10-48 hours, preferably 15-30 hours, more preferably 20-30 hours, most preferably 24 hours.

In another embodiment of the present invention, the fermentation is performed for a period of 10-48 hours, preferably 15-30 hours, more preferably 20-30 hours, most preferably 24 hours.

In another embodiment of the present invention, the enzymatic hydrolysis and/or microbial fermentation are conducted at a pH between 4.0 and 8.5, between 4.0 and 6.0, preferably between 4.5 and 5.5.

In yet another embodiment, the enzymatic hydrolysis is performed at a pH in the range of 3-7, preferably 4-6, more preferably 4.5-5.5, most preferably 5.

Step iv) is an optional separation, where the bioliquid is separated from the non-degradable solids. The separation in step iv) may be performed by any means known in art, such as in a mechanical filter, sieves, ballistic separator, washing drums, hydraulic presses, etc. In one embodiment the separation is performed before the enzymatic treatment.

Step iv) can be conducted in one separation operation or in a combination of at least two different separation operations.

A second aspect of the present invention relates to the use of a cellulosic material for producing a bioliquid suitable as a biogas substrate comprising the steps of:

(i) adding a stream of cellulosic material, such as paper, cardboard, pizza trays, carton, newspapers, magazines, office paper, diapers, pulp or paper sludge, to a bioreactor and optionally add liquid, wherein the cellulosic material has a dry matter content of cellulose of at least 20% by dry weight;

(ii) further add an enzyme comprising one or more cellulase enzymes selected from the group comprising an endoglucanase, a cellobiohydrolase, and a beta-glucosidase; followed by

(iii) enzymatic hydrolysis of the cellulosic material within the bioreactor at a temperature in the range of 35-75 °C and pH 4-7 for 2-48 hours resulting in liquefaction of the biodegradable organic matter and the cellulosic material to produce a bioliquid; optionally followed by

(iv) separation of liquid and solid; optionally followed by

(v) anaerobic fermentation of the bioliquid into a biogas.

A third aspect of the present invention relates to a biogas substrate obtained from a cellulosic material obtained by the process of:

(i) adding a stream of cellulosic material, such as paper, cardboard, pizza trays, carton, newspapers, magazines, office paper, diapers, pulp or paper sludge, to a bioreactor and optionally add liquid, wherein the cellulosic material has a dry matter content of cellulose of at least 20% by dry weight;

(ii) further add an enzyme comprising one or more cellulase enzymes selected from the group comprising an endoglucanase, a cellobiohydrolase,; followed by

(iii) enzymatic hydrolysis of the cellulosic material within the bioreactor at a temperature in the range of 35-75 °C and pH 4-7 for 2-48 hours resulting in liquefaction of the biodegradable organic matter and the cellulosic material to produce a bioliquid; optionally followed by

(iv) separation of liquid and solid; optionally followed by

(v) anaerobic fermentation of the bioliquid into a biogas.

The bioliquid obtain be the method of the invention may be subjected to Anaerobic digestion (AD), e.g., to produce methane gas, ethanol or other. AD is a series of biological processes in which microorganisms break down biodegradable material in the absence of oxygen. One of the end products is methane, which can be combusted to generate electricity, heat and/or can be processed into renewable natural gas and/or transportation fuels. A range of anaerobic digestion technologies exists in the state of the art for converting waste, such as municipal solid waste, municipal waste-water solids, food waste, high strength industrial waste water and residuals, fats, oils and grease, and various other organic waste streams into biogas. Many different anaerobic digester systems are commercially available, and the skilled person will be familiar with how to apply and optimize the anaerobic digestions process. The metabolic dynamics of microbial communities engaged in anaerobic digestion are complex. In typical anaerobic digestion for production of methane biogas, biological processes mediated by microorganisms achieve four primary steps - hydrolysis of biological macromolecules into constituent monomers, polymers and/or oligomers or other metabolites; acidogenesis, whereby short chain hydrocarbon acids and alcohols are produced; acetogenesis, whereby available nutrients are catabolized to acetic acid, hydrogen and carbon dioxide; and methanogenesis, whereby acetic acid and hydrogen are catabolized by specialized archaea to methane and carbon dioxide.

The anaerobic digestion may comprise one or more reactors operated under controlled aeration conditions, eliminating, or minimizing the available oxygen, in which methane gas is produced in each of the reactors comprising the system. The AD reactor(s) can, but need not, be part of the same waste or cellulosic material processing plant as the bioreactor in step iii) and can, but need not, be connected to the bioreactor in step iii). Moreover, the AD process may be in the form of a fixed filter system. A fixed filter anaerobic digestion system is a system in which an anaerobic digestion consortium is immobilized, optionally within a biofilm, on a physical support matrix.

In order for the AD process to work efficiently, the pH should generally remain between 6.0 and 9.0, preferably between 6.5 and 8.3. This can be largely affected by the carbon dioxide produced within the methane. The process itself produces the pH buffer (alkalinity concentration) by the production/release of $HCO_3^-$ and $NH_4^+$.

Stability may be increased by maintaining high concentrations of alkali. Decreases in pH may be due to accumulation of organic acid intermediates, often due to the presence of wastes that reduce the ability of methanogens to turn those wastes into biogas, because of the inhibition of the methanogenic conversion of previous process products into biogas. Ammonia is passively released as proteins are broken down. Bicarbonates are the primary buffer for balancing alkalinity with pH. Bicarbonate is produced in the same process as methane. Ammonia ions can be released into the liquid from protein breakdown. Ammonia is always as an equilibrium of ammonia to ammonium-ion in a liquid. When temperature increases, more is available as free ammonia, which can act as a methanogenic inhibitor at the right concentration.

To ensure proper pH maintenance, e.g., in laboratory batch digesters, alkaline agent(s) can be added at the beginning of the digestion batch. Common alkaline additives include sodium bicarbonate, potassium bicarbonate, potassium carbonate, sodium nitrate, and anhydrous ammonia. The AD digestate released from the AD process is accordingly alkaline, typically with a pH around 8.

Thus, in one embodiment of the present invention, the anaerobic digestion is conducted at a pH between 6.0 and 9.0, preferably between 6.5 and 8.5, most preferably 8.0.

In another embodiment, the anaerobic digestion is conducted within the temperature range of 30-55 °C, preferably 37-52 °C, most preferably 40-45 °C. The anaerobic digestion may be performed for a period of 5 - 30 days, preferably 10-20 days, most preferably 10-15 days.

It should be noted that embodiments and features described in the context of one of the aspects of the present invention also apply to the other aspects of the invention.

**Definitions**

Prior to discussing the present invention in further details, the following terms and conventions will first be defined: "%" In the context of the present invention, unless indicated otherwise, "%" indicates % weight/weight (w/w).

**"Aerobic"** means in the presence of free oxygen. The aerobic microbial populations proliferating in the bioliquid is accordingly able to live, being active and occur under conditions where free oxygen is present. Aerobic microorganisms have different levels of sensitivity to absence of oxygen. In the context of the present invention, aerobic microbial populations refer to microbial populations that are not capable of growth and of producing bioliquid subject to conditions absent to free oxygen.

**"Anaerobic"** means, in the context of the present invention, absence of free oxygen. The anaerobic microbial populations providing the hydrogen gas is accordingly able to live, being active and occur under conditions where free oxygen is absent. Anaerobic microorganisms have different levels of sensitivity oxygen. In the context of the present invention, anaerobic microbial populations refers to microbial populations that are not capable of growth and of producing hydrogen gas subject to conditions where free oxygen is present.

**"Anaerobic digester digestate"** or **"AD digestate"** is defined as the residual output from an anaerobic digestion (AD) used for biogas production and for removal of organic content from bioliquid. Anaerobic digesters sustainably treat organic waste e.g., from municipal, industrial, and/or agricultural operations with microorganisms under anaerobic conditions for production of biogas. Usually, the "AD digestate" has alkaline pH, and comprises low water content, non-degradable organics, suspended solids and dissolved matter such as dissociated organic and/or inorganic salts.

**"Anaerobic digestion system"** or **"AD system"** refers to a fermentation system comprising one or more reactors operated under anaerobic conditions in which methane gas is produced in each of the reactors. Methane gas is produced to the extent that the concentration of dissolved methane in the aqueous phase of the fermentation mixture within the "AD system" is saturated at the conditions used and methane gas is emitted from the system. The "AD system" may be a fixed filter system. A "fixed filter AD system" refers to a system in which an anaerobic digestion microbial consortium is immobilized, optionally within a biofilm, on a physical support matrix.

**"Biodegradables"** refers to the components in a fraction, which can be degraded biologically using enzymes and/or microorganisms.

**"Bioliquid"** is the liquefied and/or saccharified degradable components obtained by enzymatic treatment of contaminated cellulosic material from waste comprising organic matter. Bioliquid also refers to the liquid fraction obtained by enzymatic treatment of waste comprising organic matter once separated from non-fermentable solids. Bioliquid comprises water and organic substrates such as protein, fat, galactose, mannose, glucose, xylose, arabinose, lactate, acetate, ethanol and/or other components, depending on the composition of the waste (the components such as protein and fat can be in a soluble and/or insoluble form). Bioliquid comprises also fibres, ashes and inert impurities. The resulting bioliquid comprising a high percentage of solubles provides a substrate for gas production, a substrate suitable for anaerobic digestion e.g. for the production of biogas.

**"Cardboard"** means in the context of the present invention a material made from cellulose fiber, such as wood pulp, similar to paper but thicker produced by mechanically or chemically processing cellulose fibres into pulp derived from wood, rags, grasses, or other vegetable sources in water, draining the water through a fine mesh leaving the fibre evenly distributed on the surface, followed by pressing and drying. Cardboard includes regular cardboard, corrugated cardboard, pizza trays, carton, cups for cold and hot beverages etc,

**"Cellulase(s)"** according to the present invention is meant to comprise one or more enzymes capable of degrading cellulose and/or related compounds. Cellulase can also be used for any mixture or complex of various such enzymes, that act serially or synergistically to decompose cellulosic material. Cellulases break down the cellulose molecule into monosaccharides ("simple sugars") such as glucose, and/or shorter polysaccharides and oligosaccharides. Specific reactions may comprise hydrolysis of the 1,4-beta-D-glycosidic linkages in cellulose, hemicellulose, lichenin, and cereal beta-D-glucans. Several different kinds of cellulases are known, which differ structurally and mechanistically. Synonyms, derivatives, and/or specific enzymes associated with the name "cellulase" comprise endo-1,4-beta-D-glucanase (beta-1,4-glucanase, beta-1,4-endoglucan hydrolase, endoglucanase D, 1,4-(1,3,1,4)-beta-D-glucan 4-glucanohydrolase), carboxymethyl cellulase (CMCase), avicelase, celludextrinase, cellulase A, cellulosin AP, alkali cellulase, cellulase A 3, 9.5 cellulase, and pancellase SS.

Cellulases according to the present invention can also be classified based on the type of reaction catalysed, where endocellulases (EC 3.2.1.4) randomly cleave internal bonds at amorphous sites that create new chain ends, exocellulases or cellobiohydrolases (EC 3.2.1.91) cleave two to four units from the ends of the exposed chains produced by endocellulase, resulting in tetra-, tri-or disaccharides, such as cellobiose. Exocellulases are further classified into type I - that work processively from the reducing end of the cellulose chain, and type II - that work processively from the nonreducing end. Cellobiases (EC 3.2.1.21) or beta-glucosidases hydrolyse the exocellulase product into individual monosaccharides. Oxidative cellulases depolymerize cellulose by radical reactions, as for instance cellobiose dehydrogenase (acceptor). Cellulose phosphorylases depolymerize cellulose using phosphates instead of water. The prevalent understanding of the cellulolytic system divides the cellulases into three classes; **endo-1,4-[beta]-D-glucanases (EG)** (EC 3.2.1.4), which hydrolyse internal p-1,4-glucosidic bonds randomly in the cellulose chain, **exo-1,4-[beta]-D-glucanases or cellobiohydrolases (CBH)** (EC 3.2.1.91), which cleave off cellobiose units from the ends of cellulose chains;; **1,4-[beta]-D-glucosidase (EC 3.2.1.21),** which hydrolyses cellobiose to glucose and also cleaves off glucose units from cellooligosaccharides.

A commercially available cellulase preparation optimized for biomass conversion can be used, such as one that is e.g., provided by GENENCOR™ (now DuPont), DSM or NOVONESIS™.

Usually, such compositions comprise cellulase(s) and/or hemicellulase(s), such as one or more of exoglucanases, endoglucanases, endoxylanases, xylosidases, acetyl xylan esterases and beta glucosidases, including any combination thereof.

Such enzymes can e.g., be isolated from fermentations of genetically modified *Trichoderma reesei,* such as, for example, the commercial cellulase preparation sold under the trademark ACCELLERASE TRIO™ from DuPont (and/or GENENCOR). A commercially available cellulase preparation optimized for biomass conversion that can be used is provided by NOVONESIS™ and comprises exoglucanases, endoglucanases, endoxylanases, xylosidases, acetyl xylan esterases and beta glucosidases, such as, for example, the commercial cellulase preparations sold under either of the trademarks Cellic® CTec3 from NOVONESIS™.

**"Commercially available cellulase preparation optimized for biomass conversion"** refers to a commercially available mixture of enzyme activities which is sufficient to provide enzymatic treatment of biomass such as lignocellulosic biomass and which usually comprises endocellulase (endoglucanase), exocellulase (exoglucanase), endoxylanase, acetyl xylan esterase, xylosidase and/or beta-glucosidase activities.

The term "optimized for biomass conversion" refers to a product development process in which enzyme mixtures have been selected and/or modified for the specific purpose of improving yields and/or reducing enzyme consumption in treatment of biomass to fermentable sugars.

A commercially available cellulase preparation optimized for biomass conversion can be used, such as one that is e.g. provided by GENENCOR™ (now DuPont), DSM or NOVONESIS™.

Usually, such compositions comprise cellulase(s) and/or hemicellulase(s), such as one or more of exoglucanases, endoglucanases, endoxylanases, xylosidases, acetyl xylan esterases and beta glucosidases, including any combination thereof.

Such enzymes can e.g., be isolated from fermentations of genetically modified Trichoderma reesei, such as, for example, the commercial cellulase preparation sold under the trademark ACCELLERASE TRIO™ from DuPont (and/or GENENCOR). A commercially available cellulase preparation optimized for biomass conversion that can be used is provided by NOVONESIS™ and comprises exoglucanases, endoglucanases, endoxylanases, xylosidases, acetyl xylan esterases and beta glucosidases, such as, for example, the commercial cellulase preparations sold under either of the trademarks Cellic® CTec2 or Cellic® CTec3 from NOVONESIS™.

**"Cellulosic material"** means cellulose-comprising material, such as paper, cardboard, pizza trays, carton, newspapers, magazines, beverage cartons, office paper, diapers, pulp or paper sludge, material isolated from waste, such as municipal solid waste or sludge, such as pulper or paper sludge, sludge from wastewater treatment plant, wherein the cellulosic material has a dry matter content of cellulose of at least 20% by dry weight. An example of source could be any grade of source segregated paper and cardboard, source segregated milk cartoons, diapers on any other waste that contains cellulose.

**"Contaminated cellulosic material"** means cellulosic material that is contaminated with biodegradable, and/or inert and/or non-biodegradable material, such as food waste, biogenic material, plastics, sand, metal, combustible non-biodegradable material, combustible bio-degradable material. An example of contaminated material is cross contaminated segregated paper and cardboard, cross contaminated or partly wet or any other source of cellulosic material, cellulosic material that is collected in plastic bags etc.

**"Chemical Oxygen Demand"** or **"COD"** quantifies the amount of oxygen required for the total oxidation of organic material. The test does not differentiate between biologically biodegradable and non-degradable organic matter; hence, it gives an overestimate of the biogas potential of a given sample.

**"Dry matter,"** also appearing as "DM", refers to total solids, both soluble and insoluble, and effectively means "non-water content." Dry matter content is measured by drying at approximately between 60 to 105°C until constant weight is achieved. In a preferred embodiment, dry matter content is measured by drying at approximately 105 °C. The lower temperature range is used when the analysis substrate contains volatile compounds which may escape when boiling water and decrease the analysis result accuracy.

**"Fermenting microorganism"** refers to any microorganism, including bacterial and fungal organisms, suitable for use in a desired fermentation process to produce a fermentation product. The fermenting organism can be hexose and/or pentose fermenting organisms, or a combination thereof. Both hexose and pentose fermenting organisms are well known in the art. Suitable fermenting microorganisms are able to ferment, i.e., convert, sugars, such as glucose, xylose, xylulose, arabinose, maltose, mannose, galactose, and/or oligosaccharides, directly or indirectly into the desired fermentation product. The fermenting microorganism can e.g. produce glucose based chemicals, such as lactic acid, 3-hydroxypropionic acid (3-HPA), 1,4-butanediol (BDO), butanedioic acid (succinic acid), ethane-1,2-diol (ethylene glycol), butanol and/or 1,2-propanediol (propylene glycol). Examples of fermenting yeast include strains of *Candida, Kluyveromyces,* and *Saccharomyces,* such as Candida sp., e.g. *Candida sonorensis, Kluyveromyces sp., e.g. Kluyveromyces marxianus,* and *Saccharomyces sp., e.g. Saccharomyces cerevisiae.*

Examples of fermenting organisms that can ferment pentose sugars in their native state include bacterial and fungal organisms, such as some yeast. Xylose fermenting yeast include strains of *Candida,* preferably *C. sheatae* or *C. sonorensis;* and strains of *Pichia,* e.g., *P. stipitis,* such as *P. stipitis* CBS 5773. Pentose fermenting yeast include strains of *Pachysolen,* preferably *P. tannophilus.* Organisms not capable of fermenting pentose sugars, such as xylose and arabinose, may be genetically modified to do so by methods known in the art.

**"Hydrolysis"** is the splitting of chemical bond with the participation of water as co-substrate. The term is applied when municipal solid waste material is treated with an enzyme to break down cellulose and/or hemicellulose and other substrates to fermentable sugars, such as glucose, cellobiose, xylose, xylulose, arabinose, mannose, galactose, and/or soluble oligosaccharides (also known as saccharification). The enzymatic treatment is performed enzymatically by one or more enzymes in one or more stages. In the present disclosure, the terms "hydrolyzation", "liquefaction", "saccharification" and "solubilization" may be used interchangeably.

**"Inoculum"** the incoming MSW stream may simply be inoculated with an inoculum of microorganisms naturally occurring in the waste, and optionally "raised" on local waste or components of local waste as a food source in fermentation conditions of temperature within the range 37 to 55°C, or 40 to 55°C, or 45 to 50°C, and at a pH within the range 4.2 and 6.0.

**"Lactic acid producing bacteria"** comprises lactic acid bacteria (LAB) where the currently accepted taxonomy is based on the List of Prokaryotic names with Standing in Nomenclature (LPSN) - an online database that maintains information on the naming and taxonomy of prokaryotes, following the taxonomy requirements and rulings of the International Code of Nomenclature of Bacteria. The phylogeny of the order is based on 16S rRNA-based LTP release 106 by 'The All-Species Living Tree' Project. In addition to bacteria belonging to the LAB order, the term "lactic acid producing bacteria" used herein also comprises bacteria that do not belong to the LAB order, but that are nevertheless capable of producing lactic acid.

**"Liquid fraction",** means the mainly liquid slurry obtained after the waste to be processed has been subjected to a combined enzymatic and microbial treatment and thereafter has been subjected to one or more separation step(s), separating the treated waste into a liquid, i.e. a slurry, and a solid or semi-solid fraction.

**"Methane Potential test"** refers to a biological test providing a fast indication of the methane production that can be achieved by anaerobic digestion. It is expressed e.g. in l $CH_4$/kg MSW/cellulosic material, or mL-CH4/g-VS.

**"Municipal solid waste"** (MSW) refers to waste fractions which are typically available in a city, but that need not come from any municipality *per se,* i.e., MSW refers to every solid waste from any municipality but not necessarily being the typical household waste - could be waste from airports, universities, campus, canteens, general food waste, among others. The terms municipal solid waste and household waste may be used interchangeably in this application. MSW may be any combination of one or more of cellulosic, plant, animal, plastic, metal, or glass waste including, but not limited to, any one or more of the following: Garbage collected in normal municipal collections systems, optionally processed in a central sorting, shredding or pulping device, such as e.g., a Dewaster® or a reCulture®; solid waste sorted from households, including both organic fractions and paper rich fractions; Generally, municipal solid waste in the Western part of the world normally comprise one or more of: animal food waste, vegetable food waste, newsprints, magazines, advertisements, books, office paper, other clean paper, paper and carton containers, other cardboard, milk cartons and alike, juice cartons and other carton with alu-foil, kitchen tissues, other dirty paper, other dirty cardboard, soft plastic, plastic bottles, other hard plastic, non-recyclable plastic, yard waste, flowers etc., animals and excrements, diapers and tampons, cotton sticks etc., other cotton etc., wood, textiles, shoes, leather, rubber etc., office articles, empty chemical bottles, plastic products, cigarette buts, other combustibles, vacuum cleaner bags, clear glass, green glass, brown glass, other glass, aluminium containers, alu-trays, alu-foil (including tealight candle foil), metal containers (-Al), metal foil (-Al), other sorts of metal, soil, rocks, stones and gravel, ceramics, cat litter, batteries (button cells, alkali, thermometers etc.), other non-combustibles and fines.

**"Paper"** means in the context of the present invention a sheet material produced by mechanically or chemically processing cellulose fibres into pulp derived from wood, rags, grasses, or other vegetable sources in water, draining the water through a fine mesh leaving the fibre in the form of pulp evenly distributed on the surface, followed by pressing and drying.

Pulp can be prepare from wood using a chemical pulping process separates lignin from cellulose fibre. A cooking liquor is used to dissolve the lignin, which is then washed from the cellulose. Removal of lignin preserves the length of the cellulose fibres. Paper includes regular paper, newspapers, magazines, office paper, pulp or paper sludge, etc.

**"Paper sludge"** or **"Pulp sludge"** is the main organic residual generated from the wastewater treatments of the pulp and paper industry. Thus, pulp and paper sludge from paper mills are the organic residual material remaining after wastewater treatment in the pulp and paper mills, which contain fibres which are too short for paper production, holds a biogas potential, which are currently not harvested or utilized.

**"Reject water"** is defined as the liquid fraction obtained after one or more solid-liquid separations of the digestate from anaerobic digestion of bioliquid obtained form the solid/liquid separation of the liquefied cellulosic material. The one or more solid liquid separations can comprise one or more of decantation, centrifugation, filtering, flocculation, pressing and sedimentation. Reject water has an alkaline pH and comprises dissolved matter, such as salts, which may include both inorganic salts and organic salts. Reject water may also comprise some suspended matter and live microorganisms from the AD process.

**"Sorted",** refers to a process in which waste, such as MSW, is substantially fractionated into separate fractions such that organic material is substantially separated from plastic and/or other non-biodegradable material.

**"Sorted waste"** (or "sorted MSW") as used herein refers to waste in which approximately less than 30%, preferably less than 20% and most preferably less than 15% by weight of the dry weight is not biodegradable material.

**"Total Solids"** or **"TS"** is a measure of the material remaining after removal of water at 60°C or 100°C. The lower temperature ensures that volatile organic compounds are not lost and accounted for as water. The higher temperature is used for some fractions where loss of volatiles is not considered an issue.

**"Unsorted"** refers to that the waste or the MSW is not substantially fractionated into separate fractions such that organic material is not substantially separated from plastic and/or other inorganic material, notwithstanding removal of some large objects or metal objects and notwithstanding some separation of plastic and/or other inorganic material may have taken place. The terms "unsorted waste" (or "unsorted MSW"), as used herein, refers to waste comprising a mixture of biodegradable and non-biodegradable material in which 15% by weight or greater of the dry weight is non-biodegradable material. Waste that has been briefly sorted yet still produce a waste (or MSW) fraction that is unsorted. Typically, unsorted MSW may comprise organic waste, including one or more of food and kitchen waste; paper- and/or cardboard-containing materials; recyclable materials, including glass, bottles, cans, metals, and certain plastics; burnable materials; and inert materials, including ceramics, rocks, and debris. The recyclable material might be before or after source sorting.

**"Waste"** comprises, sorted and unsorted, municipal solid waste (MSW), agriculture waste, hospital waste, industrial waste, e.g., waste fractions derived from industry such as restaurant industry, food processing industry, general industry; waste fractions from paper industry; waste fractions from recycling facilities; waste fractions from food or feed industry; waste fraction from the medicinal or pharmaceutical industry; waste fractions from hospitals and clinics, waste fractions derived from agriculture or farming related sectors; waste fractions from processing of sugar or starch rich products; contaminated or in other ways spoiled agriculture products such as grain, potatoes and beets not exploitable for food or feed purposes; or garden refuse.

**"Waste fractions derived from agriculture or farming related sectors"** comprises waste fractions from processes including sugar or starch rich products such as potatoes and beet; contaminated or in other ways spoiled agriculture products such as grain, potatoes and beet not exploitable for food or feed purposes; garden refuse; manure, or manure derived products.

**"Waste fractions derived from households"** comprises unsorted municipal solid waste (MSW); MSW processed in some central sorting, shredding or pulping device such as e.g. Dewaster® or reCulture®; Solid waste sorted from households, including both organic fractions and paper rich fractions; RDF (Refuse-Derived-Fuel); fraction derived by post treatment as e.g. inerts, organic fractions, metals, glass, and plastic fractions. In a preferred embodiment a 2D and 3D fraction is prepared. The 2D fraction can be further separated into recyclables and/or residuals such as SRF (Solid Recovered Fuel), RDF (Refused Derived Fuel) and/or inerts. The 3D fraction can also be further separated into recyclables and/or residuals such as metals, 3D plastic and/or RDF.

**"Waste fractions derived from the industry"** comprises general industry waste fractions containing paper, cardboard or other organic fractions; waste fraction from paper industry, e.g. from recycling facilities; waste fractions from food and feed industry; waste fractions from the medicinal industry, hospital and clinic waste, airport waste, other public and private services derived waste.

It should be noted that embodiments and features described in the context of one of the aspects of the present invention also apply to the other aspects of the invention.

All patent and non-patent references cited in the present application, are hereby incorporated by reference in their entirety.

The invention will now be described in further details in the following non-limiting examples.

**Items**

    1. A method for processing cellulosic material, wherein the method comprises the steps of:

(i) adding a stream of cellulosic material, such as paper, cardboard, pizza trays, carton, newspapers, magazines, office paper, diapers, pulp or paper sludge, to a bioreactor and optionally add liquid, wherein the cellulosic material has a dry matter content of cellulose of at least 20% by dry weight;

(ii) further add an enzyme comprising one or more cellulase enzymes selected from the group comprising an endoglucanase, a cellobiohydrolase, and a beta-glucosidase and one or more enzymes selected from the list comprising a protease, a lipase, a beta-glucanase, a pectate lyase, a hemicellulase, a mannanase, and an amylase; followed by

(iii) enzymatic hydrolysis of the cellulosic material within the bioreactor at a temperature in the range of 35-75 °C and pH 4-7 for 2-48 hours resulting in liquefaction of the biodegradable organic matter and the cellulosic material to produce a bioliquid; optionally followed by

(iv) separation of liquid and solid; optionally followed by

(v) anaerobic fermentation of the bioliquid into a biogas.

2. The method according to item 1, wherein the cellulosic material is contaminated with biodegradable, and/or inert and/or non-biodegradable material, such as food waste, biogernic material, plastics, sand, metal, non-biodegradable combustible material, material isolated from waste, such as municipal solid waste or sludge, such as pulper or paper sludge, sludge from wastewater treatment plant.

3. The method of item 2, wherein the contaminated cellulosic material comprises between 20 and 99% by weight cellulosic material, preferably between 30 and 80% by weight cellulosic material, more preferably between 40 and 70% by weight cellulosic material of the treated stream.

4. The method of any of the preceding items, wherein the cellulosic material comprises between 30 and 99% by dry weight cellulose, preferably between 30 and 80% by weight cellulose, more preferably between 35 and 60% by weight cellulose on a dry matter basis.

5. The method of any of the preceding items, wherein the cellulosic material comprises between 0 and 40% by weight hemicellulose, preferably between 1 and 25% by weight hemicellulose, more preferably between 5 and 25% by weight hemicellulose on a dry matter basis and/or wherein the cellulosic material comprises between 0 and 30% by weight lignin, preferably between 0 and 25% by weight lignin, more preferably between 1 and 25% by weight, most preferably between 5 and 25% by weight lignin on a dry matter basis.

6. The method of any of the preceding items, wherein the cellulosic material is preferably mechanically treated such as shredded before the enzymatic hydrolysis, preferably by cutting the cellulosic material into smaller pieces, such as having a screen size between 30 mm to about 400 mm, preferably a screen size between 40 mm to 250 mm, more preferably a screen size between 40 mm to 150 mm, most preferably 100 mm or below.

7. The method according to any of the proceeding items, wherein the enzymes are added as recirculated bioliquid, process water, microorganism secreting enzyme, or as an enzyme composition.

8. The method of any of the preceding items, wherein the cellobiohydrolase is one or more enzymes selected from the group comprising a cellobiohydrolase I, a cellobiohydrolase II, and a beta-glucosidase.

9. The method of any of the preceding items, wherein the enzyme further comprises one or more enzymes selected from an AA9 polypeptide, a cellulose inducible protein (CIP), an esterase, an expansin, a ligninolytic enzyme, an oxidoreductase, a pectinase, and a swollenin.

10. The method of any of the preceding items, wherein the hemicellulase is one or more enzymes selected from a xylanase, an acetylxylan esterase, a feruloyl esterase, an arabinofuranosidase, a xylosidase, and a glucuronidase.

11. The method of any of the preceding items, wherein the enzymatic hydrolysis is performed at a temperature in the range of 40-60 degrees C, more preferably 45-55 degrees C, most preferably 50 degrees C.

12. The method of any of the preceding items, wherein the enzymatic hydrolysis is performed at a pH in the range of 4-6, more preferably 4.5-5.5, most preferably 5.

13. The method of any of the preceding items, wherein the enzymatic hydrolysis is performed for a period of wherein the enzymatic hydrolysis is performed for a period of 10-48 hours, preferably 15-30 hours, more preferably 20-30 hours, most preferably 24 hours.

14. The method of any of the preceding items, wherein the cellulosic material is pretreated for 3 hours in water at 50-60 degrees C.

15. The method of any of the preceding items, wherein the cellulosic material is subjected to fermentation concomitantly with or after the enzymatic hydrolysis to further liquify the cellulosic material.

16. The method of any of the preceding items, wherein the anaerobic fermentation is conducted at a temperature in the range of 30-75 degrees C, preferably 40-60 degrees C, more preferably 45-55 degrees C, most preferably 50 degrees C.

17. The method of any of the preceding items, wherein the anaerobic fermentation is performed for a period of 10-48 hours, preferably 15-30 hours, more preferably 20-30 hours, most preferably 24 hours.

18. The method of any of the preceding items, wherein the methane potential of the liquefied cellulosic material is 25-250 L/kg dry cellulosic material, preferably 50-250 L/kg cellulosic material, more preferably 100-250 L/kg dry cellulosic material and most preferably 150-250 L/kg dry cellulosic material.

19. The method of any of the preceding items, wherein

the anaerobic fermentation in step (iv) is conducted by transferring the bioliquid into an anaerobic digestion system; followed by

conducting anaerobic fermentation of the bioliquid to produce biogas, and wherein the anaerobic fermentation is conducted at a pH between 6.0 and 9.0, preferably between 6.5 and 8.5, most preferred 8.0.

20. Use of a cellulosic material for producing a bioliquid suitable as a biogas substrate comprising the steps of:

(i) adding a stream of cellulosic material, such as paper, cardboard, pizza trays, carton, newspapers, magazines, office paper, diapers, pulp or paper sludge, to a bioreactor and optionally add liquid, wherein the cellulosic material has a dry matter content of cellulose of at least 20% by dry weight;

(ii) further add an enzyme comprising one or more cellulase enzymes selected from the group comprising an endoglucanase, a cellobiohydrolase, and a beta-glucosidase and one or more enzymes selected from the list comprising a protease, a lipase, a beta-glucanase, a pectate lyase, a hemicellulase, a mannanase, and an amylase; followed by

(iii) enzymatic hydrolysis of the cellulosic material within the bioreactor at a temperature in the range of 35-75 °C and pH 4-7 for 2-48 hours resulting in liquefaction of the biodegradable organic matter and the cellulosic material to produce a bioliquid; optionally followed by

(iv) separation of liquid and solid; optionally followed by

(v) anaerobic fermentation of the bioliquid into a biogas.

21. A biogas substrate obtained from a cellulosic material obtained by the process of:

(i) adding a stream of cellulosic material, such as paper, cardboard, pizza trays, carton, newspapers, magazines, office paper, diapers, pulp or paper sludge, to a bioreactor and optionally add liquid, wherein the cellulosic material has a dry matter content of cellulose of at least 20% by dry weight;

(ii) further add an enzyme comprising one or more cellulase enzymes selected from the group comprising an endoglucanase, a cellobiohydrolase, and a beta-glucosidase and one or more enzymes selected from the list comprising a protease, a lipase, a beta-glucanase, a pectate lyase, a hemicellulase, a mannanase, and an amylase; followed by

(iii) enzymatic hydrolysis of the cellulosic material within the bioreactor at a temperature in the range of 35-75 °C and pH 4-7 for 2-48 hours resulting in liquefaction of the biodegradable organic matter and the cellulosic material to produce a bioliquid; optionally followed by

(iv) separation of liquid and solid; optionally followed by

(v) anaerobic fermentation of the bioliquid into a biogas.

**Examples**

Introduction

To assess cellulosic fractions experimentally, the following approach was chosen. Initially, several fractions were selected to represent the real-world fractions, which can be found in the waste. Subsequently, these fractions were enzymatically hydrolyzed in fermenters under different experimental conditions to determine the release of organic material. Also, several preprocessing techniques were applied to the fractions before the hydrolysis to evaluate the effects of these techniques on hydrolysis efficiency. Finally, the hydrolyzed fractions were treated in AMPTS setups (The Automatic Methane Potential Test System) to measure their biomethane potential and evaluate how the type of the fractions as well as hydrolysis and pretreatment settings affected the methane production.

This approach provides an estimate of the biomethane potential for each individual fraction. Subsequently, this can be used to calculate and estimate the biomethane potential of a mixture of the used fractions based on the composition analysis of a cellulosic waste of interest.

The following 5 types of cellulosic fractions were used in the current study:

- Clear office paper **(Fejl! Henvisningskilde ikke fundet.** top row). This fraction represents high quality paper that can be found in the waste and is expected to have the highest biomethane potential.
- Corrugated brown cardboard **(Fejl! Henvisningskilde ikke fundet.** second row, left). This fraction represents cardboard fractions (which are often encountered in waste). Corrugated cardboard has normally a lower quality than the office paper, however, it still has a relatively high amount of sugar polymers, which is why it is expected to have a relatively high biomethane potential.
- Newspapers **(Fejl! Henvisningskilde ikke fundet.** third row). This fractions together with the magazines fraction represent a lower quality cellulosic fraction in waste. These fractions normally consist of lower grade paper which is why it is expected to have a relatively low biomethane potential.
- Magazines **(Fejl! Henvisningskilde ikke fundet.** last row)
- Milk cartons **(Fejl! Henvisningskilde ikke fundet.** second row, right).

This fraction represents different types of juice and milk cartons found in waste.

**Analysis of Cellulosic Fractions Used in the Examples**

The composition of the fractions was analyzed by using a strong acid hydrolysis procedure and the results are summarized in **Fejl! Henvisningskilde ikke fundet.**

The analysis demonstrated that paper and cardboard contained a similar amounts of sugar polymers (cellulose and xylose) and the difference in the composition is due to lignin and ash. Magazine, newspaper and milk carton fractions contained noticeably less sugars and much more ash and lignin. This is expected because newspapers and magazines are normally made of a lower grade paper and in addition contain ink and different coating. Milk cartons consist of several layers of plastic and paper which is why the strong acid hydrolysis yielded a high amount of lignin (plastic is attributed as lignin by this acid hydrolysis method) and a relatively low amount of cellulose and xylan.

Comparing the obtained results with the literature composition for office paper, cardboard, newspapers and magazines (**Fejl! Henvisningskilde ikke fundet.**), one can see that the fractions used in the current studies are close in composition to the reported ones (Wang 2012, Chu 2013, Elliston 2013, Ioelovich 2014, Zhou 2017). This in turn means that the used fractions are representative, and the results obtained further in these studies with these fractions are expected to be similar for any kind of office paper, cardboard, newspapers and magazines.

**Experiments in Fermenters**

All the enzymatic hydrolysis experiments were conducted in 1-L fermenters using a cellulase enzyme, at a temperature of 50 °C, with 1,1 L of added liquid as the reaction media (tap water or acidified reject water). 0-60 g of a fraction was used; several experiments were set up with model waste. For most of the experiments the pH was maintained around 5 by addition of 4 M hydrochloric acid or sodium hydroxide during the fermentation procedure, few experiments were conducted without any pH adjustment. All the experimental setups are described in
Table 1. To follow the progress of the fermentations, samples ¨were collected at 0, 2, 18 and 24 h after the initiation of the experiment. Samples were analyzed by HPLC and dissolved COD determinations. When acidified reject water was used, raw reject water was first acidified to pH 5.7 by using acetic acid and then was added to the fermenters where pH was further adjusted to 5 as described above.

To allow comparison of results from different fermentations, all concentrations were converted from g/L to g/g fraction TS. This was required because the volume of each individual fermentation was slightly different from all other fermentations and in addition was not constant during the course of the experiment. All this is due to the pH adjustment and different volume of HCl and NaOH added. In addition, to compare COD values (chemical oxygen demand) from the experiments with different enzyme doses, the contribution of the enzymes in both total and dissolved COD was subtracted (0.25 g COD/g enzymes).

## Table 1. Summary of the experimental setups for Cellulosic Fractions-02

| Run | pH | Fraction (g) | Frac type | Pre-treatment | Added waste (g) | Enzyme (g) | Used in AMPTS |
|---|---|---|---|---|---|---|---|
| 1 | 5 | 30 | Office paper | Shred | 0 | 7.2 | |
| 2 | 5 | 30 | Cardboard | Shred | 0 | 7.2 | |
| 3 | 5 | 30 | Newspaper | Shred | 0 | 7.2 | |
| 4 | 5 | 30 | Magazine | Shred | 0 | 7.2 | |
| 5 | 5 | 30 | Milk carton | Shred | 0 | 7.2 | |
| 6 | 5 | 30 | Office paper | Cut | 0 | 7.2 | |
| 7 | 5 | 30 | Newspaper | Cut | 0 | 7.2 | |
| 8 | 5 | 30 | Magazine | Cut | 0 | 7.2 | |
| 9 | 5 | 30 | Office paper | Shred +3 h in warm water | 0 | 7.2 | Yes |
| 10 | 5 | 30 | Cardboard | Shred +3 h in warm water | 0 | 7.2 | Yes |
| 11 | 5 | 30 | Newspaper | Shred +3 h in warm water | 0 | 7.2 | Yes |
| 12 | 5 | 30 | Magazine | Shred +3 h in warm water | 0 | 7.2 | Yes |
| 13 | No control | 10 | Office paper | Shred | 166 | 7.2 | |
| 14 | No control | 10 | Cardboard | Shred | 166 | 7.2 | |
| 15 | No control | 10 | Newspaper | Shred | 166 | 7.2 | |
| 16 | No control | 10 | Magazine | Shred | 166 | 7.2 | |
| 17 | No control | 0 | None | – | 166 | 7.2 | |
| 18 | No control | 10 | Milk carton | Shred | 166 | 7.2 | |
| 19 | 5 | 30 | Cardboard | Shred | 0 | 7.2 | Yes |
| 20 | 5 | 30 | Cardboard | Cut | 0 | 7.2 | Yes |
| 21 | 5 | 30 | Newspaper | Cut | 0 | 7.2 | Yes |

| Run | pH | Fraction (g) | Frac type | Pre-treatment | Added waste (g) | Enzyme (g) | Used in AMPTS |
|---|---|---|---|---|---|---|---|
| 22 | No control | 10 | Office paper | Shred | 166 | 8.7 | |
| 23 | No control | 10 | Cardboard | Shred | 166 | 8.7 | |
| 24 | No control | 10 | Newspaper | Shred | 166 | 8.7 | |
| 25 | No control | 10 | Magazine | Shred | 166 | 8.7 | |
| 26 | No control | 0 | None | – | 166 | 8.7 | |
| 27 | 5 | 60 | Cardboard | Shred | 0 | 7.2 | Yes |
| 28 | 5 | 30 | Office paper | Shred | 0 | 1 | |
| 29 | 5 | 30 | Cardboard | Shred | 0 | 1 | |
| 30 | 5 | 30 | Newspaper | Shred | 0 | 1 | |
| 31 | 5 | 30 | Magazine | Shred | 0 | 1 | |
| 32 | No control | 10 | Office paper | Shred | 150 | 7.2 | |
| 33 | No control | 10 | Cardboard | Shred | 150 | 7.2 | |
| 34 | No control | 10 | Newspaper | Shred | 150 | 7.2 | |
| 35 | No control | 10 | Magazine | Shred | 150 | 7.2 | |
| 36 | No control | 0 | None | – | 166 | 7.2 | |
| 37 | No control | 16 | Milk carton | Shred | 166 | 7.2 | |
| 38 | 5 | 30 | Office paper | Shred | 0 | 3.6 | |
| 39 | 5 | 30 | Cardboard | Shred | 0 | 3.6 | |
| 40 | 5 | 30 | Newspaper | Shred | 0 | 3.6 | |
| 41 | 5 | 30 | Magazine | Shred | 0 | 3.6 | |
| 42 | 5 | 30 | Magazine | Shred – Same as 41 | 0 | 3.6 | Yes |
| 43 | 5 | 30 | Newspaper | Shred – Same as 40 | 0 | 3.6 | Yes |
| 44 | 5 | 30 | Office paper | Shred – Same as 28 | 0 | 1 | Yes |

| Run | pH | Fraction (g) | Frac type | Pre-treatment | Added waste (g) | Enzyme (g) | Used in AMPTS |
|---|---|---|---|---|---|---|---|
| 45 | 5 | 30 | Cardboard | Shred – Same as 29 | 0 | 1 | Yes |
| 46 | 5 | 30 | Newspaper | Shred – Same as 30 | 0 | 1 | Yes |
| 47 | 5 | 30 | Newspaper | Shred | 0 | 0 | |
| 48 | 5 | 30 | Magazine | Shred | 0 | 0 | |
| 49 | 5 | 30 | Cardboard | Shred | 0 | 0 | |
| 50 | 5 | 30 | Milk carton | Shred | 0 | 0 | |
| 51 | 5 | 30 | Office paper | Shred | 0 | 0 | |
| 52 | 5 | 30 | Newspaper | Shred – Reject water | 0 | 7.2 | Yes |
| 53 | 5 | 30 | Magazine | Shred – Reject water | 0 | 7.2 | Yes |
| 54 | 5 | 30 | Cardboard | Shred – Reject water | 0 | 7.2 | Yes |
| 55 | 5 | 30 | Office paper | Shred – Reject water | 0 | 7.2 | Yes |
| 56 | 5 | 30 | Office paper | Shred | 0 | 7.2 | Yes |
| 57 | 5 | 30 | Magazine | Shred | 0 | 1 | Yes |
| 58 | 5 | 30 | Magazine | Shred | 0 | 7.2 | Yes |
| 59 | 5 | 30 | Newspaper | Shred | 0 | 7.2 | Yes |
| 60 | No control | 16 | Office paper | Shred | 150 | 7.5 | |
| 61 | No control | 16 | Cardboard | Shred | 150 | 7.5 | |
| 62 | No control | 16 | Newspaper | Shred | 150 | 7.5 | |
| 63 | No control | 16 | Magazine | Shred | 150 | 7.5 | |
| 64 | No control | 0 | None | – | 166 | 7.5 | |

The following effects were studied in the fermenters (and consequently in AMPTS):

- How pre-soaking of the fractions in warm water would affect the process. In real world, the fractions can get wet for many reasons before arriving to the plant which is why it is important to understand whether dry (i.e. ideally stored) and wet fractions would behave differently.
- How the size of the fractions would affect the process.
- Whether the hydrolysis in pure (tap) water and in acidified reject water, would affect the process.
- How different enzyme dosages would affect the process.

- Whether the cellulosic fractions would behave similarly when mixed with model waste as when hydrolyzed alone.

The effect of pre-soaking was studied as follows: a cellulosic fraction was mixed with tap water, heated to 50 °C and stirred at this temperature for 3 h, then enzymes were added which represented the 0 h time point of the experiment. It was found that for all the 4 fractions studied the pre-soaking yielded a lower dissolved COD when compared to a regular experiment (**Fejl! Henvisningskilde ikke fundet.**)**.**

The effect of fractions sizes (dimensions) was assessed as follows: the cellulosic fractions were cut into large (also referred to as "cut") and small pieces (also referred to as "shred"). The large pieces were at least 1 cm wide and 10 cm long **(Fejl! Henvisningskilde ikke fundet.** a, d, e, g) while the small pieces were at max 0.5 cm wide and 7-8 cm long **(Fejl! Henvisningskilde ikke fundet.** b, c, f, h). The hydrolysis of these fractions revealed that the larger pieces released slightly less dissolved COD that the small ones (Figure 1). To make sure that the results for all other experiments are comparable, all the other experiments (including the pre-soaking ones) were set up with small fractions.

The effect of the enzyme dosage was assessed by maintaining identical experiments conditions while varying the dose of enzymes. The results were mainly evaluated based on the total and dissolved COD with the contribution of enzymes subtracted (Figure 2, Figure 3). It was discovered that for each fraction, the dissolved COD was lower at lower enzyme dosages, however, the decrease in COD was disproportional to that of enzymes used. For example, when the enzyme dose was reduced 2 times (from 7.2 to 3.6 g) the resulting dissolved COD for cardboard was reduced 1.25 times and when the dosage was reduced further 3.6 times (from 3.6 to 1 g) the dissolved COD was reduced 1.05 times. Interestingly, total COD was reduced even less for most of the fractions when reducing the enzyme dosage. For example, for office paper the total COD was reduced 1.3 times when enzyme dose was reduced from 7.2 to 1 g.

The effect of the enzyme dosage on the total COD suggests that enzymes play two roles in the hydrolysis experiments:

- they bring a part of organic material from the cellulosic fractions (mainly sugars) into solution.
- they destroy the structure (framework) of the fractions and convert one big piece of the fraction into many tiny pieces (that are not necessarily fully dissolved) which can be detected by the total COD measurements (which can detect any particles made of organic materials which are small enough to go through a pipette tip (~1 mm) when making a dilution for the measurement).

Looking at the absolute COD values (with the contribution of enzymes subtracted) of the different fractions, it can be seen that the total COD was quite similar for the different fractions and enzyme loadings (Figure 3). This observation further supports the idea that the enzymes are responsible for breaking the structure of the fractions and suspending many tiny particles from one big piece of the fraction. At the same time, the dissolved COD was found to be different for the different fractions. This result is expected because the fractions contain rather different amount of sugar polymers (**Fejl! Henvisningskilde ikke fundet.**), which are the primary targets for the cellulase enzymes.

After having studied the enzymatic hydrolysis of the cellulosic fraction alone, the next step is to assess whether the fractions can release a similar amount of organic material when mixed with model waste. These experiments can suggest how cellulosic fractions can be treated in a full-scale plant.

Four kinds of experiments were set up where a fraction constituted up to 10 additional wt% of the model waste:

1. A fraction (10 g) was added to a regular fermentation of model waste (166 g, 7.2 g enzyme), runs 13-18 in
2. Table 1.
3. A fraction (10 g) and proportionally more enzymes were added to a regular fermentation of model waste (166 g, 7.2 g enzyme), runs 22-26 in
4. Table 1.
5. A fraction (10 g) was added to model waste (150 g) to approximately match the total weight of model waste used in a regular fermentation. Regular amount of enzyme was used (7.2 g), runs 32-37 in
6. Table 1.
7. A fraction (16 g) was added to model waste (150 g) to exactly match the total weight of model waste. Amount of enzyme was adjusted to match the same dosage relative to dry matter
8. Table 1.

The experiments were set up without any pH control and were evaluated by comparing the expected dissolved COD released from the fraction, with the actual additional dissolved COD. It was found that under almost all the tested conditions, the cellulosic fractions released some additional dissolved COD ranging between 50 and 180% of the expected COD. It was particularly interesting to find that adding a fraction to the otherwise regular fermentation experiments (case 1) led to an increase in dissolved COD for all the 4 fractions. Overall, the findings suggest that the addition of the cellulosic fractions to the fermentation of model waste can in general increase dissolved COD.

Finally, the effect of acidified reject water on the enzymatic hydrolysis of the cellulosic fractions was evaluated. In these experiments, acidified reject water was used instead of tap water with otherwise identical conditions. It was found that dissolved COD released from the fractions in acidified reject water was comparable or even slightly higher than in the experiments in tap water (Figure 5).

In conclusion, the fermenter experiments showed that the increase in size of the fractions and pre-soaking in warm water might have a small negative effect on the dissolved COD of the hydrolysis. It was also found that reducing the enzyme dosage reduces the dissolved COD while the total COD was only mildly affected for all the tested fractions. Acidified reject water did not have any negative effects of the performance of the enzymes. Finally, the addition of the cellulosic fractions to the fermentation of model waste increases the dissolved COD.

**Biogas Experiments in AMPTS**

To measure biomethane potential of the fractions enzymatically hydrolyzed in fermenters, several fractions were digested in AMPTS (automatic methane potential test systems) where methane production was measured (see the selected fractions in

Table 1, column Used in AMPTS). The experiments were set up in two batches and a total of 21 different fermenter experiments were further tested in AMPTS (each of the tested fermenter sample was analyzed in triplicates in AMPTS). Inoculum from Billund Wastewater Treatment plant was used for all the experiments: the Inoculum is sampled from the thermophilic biogas digester at Billund vand treating a mixture of WWTP primary sludge and source separated organic fraction from households (including tissues, napkins and kitchen rolls). The substrate was sampled from a fermenter at the end of the enzymatic hydrolysis experiment (24 h) by thoroughly homogenizing the mixture and pouring the contents into a 500-mL glass bottle which was immediately frozen and thawed before the AMPTS experiment. The inoculum-to-substrate VS ratio (volatile solids) in each AMPTS experiment was targeted to be around 10:1. Out of all the 75 experiments (21 substrate in triplicates, 2 blanks and 2 cellulose both in triplicates), only two bottles did not produce any methane (one blank in batch 2 and one from run 57, Table 1) while the rest yielded 50-400 mL of $CH_4$.

The methane production was converted to the specific methane production (NmL CH4/g VS) but using the following formula:

$$y_i = \frac{V_{CH4} - m_{inoc} \cdot X \cdot y^{blank}}{m_{substrate} \cdot S} \qquad (1)$$

where: $y_i$ is the specific methane production of the substrate, observed in bottle I; $y^{blank}$ is the average specific methane production of the blank, in mL $CH_4$/g VS; $V_{CH4}$ is the cumulative methane production of bottle i, expressed in mL $CH_4$; $m_{inoc}$ is the amount of inoculum added to the bottle i, in g; $X$ is the concentration of the inoculum stock solution, in g-VS/g; $S$ is the concentration of the substrate stock solution, in g VS/g; $m_{substrate}$ is the amount of substrate added to the bottle i, in g.

Maximum of the mean curve was used as the actual specific methane yield for all the experiments. An average factor for converting COD of proteins into methane of 300 NmL $CH_4$/COD was used.

In addition to the specific methane yields, the rate of methane production is another interesting result to evaluate. It was found that all the experiments with the hydrolysed fractions had the fastest rate of methane production between 0 and 50 h while after 50 h the rate went down to almost 0 NmL/h. This observation suggests that most of the easily digestible substrate in bioliquid was converted to methane within the first two days of the experiment. At the same time, several experiments had one more peak on the methane production rate curves between 50 and 100 h. Most likely, this second peak corresponds to the production of methane from cellulose which was not fully hydrolysed by enzymes in fermenters. Interestingly, there were only 4 AMPTS experiments which had a pronounced second peak at 50-100 h. These 4 experiments share a common feature, which is that the amount of enzyme relative to the amount of substrate was lower than in other experiments with the same fractions. Apparently, even though these lower enzyme loadings did not entirely hydrolyse or dissolve all the cellulose in the fraction samples, the enzymes still affected the the fractions in a way that generally opened up more rigid cellulose structures thereby making them more accessible to the biogas microbes. This observation highlights again that enzymes are important not only to hydrolyse and dissolve cellulose but also to break the structure of the cellulosic fractions.

The effect of pre-soaking on the methane production was evaluated (Figure 10) in addition to dissolved COD as discussed in the previous part (**Fejl! Henvisningskilde ikke fundet.**). While the dissolved COD showed a small negative impact of pre-soaking, the methane production was found to be almost identical for pre-soaked and non-treated fractions.

The effect of lowering enzyme dosage was also evaluated based on the methane production (Figure 10 and 11) in addition to total and dissolved COD discussed in the previous section (Figure 2 and Figure 3). Interestingly, it was discovered that while on average a lower amount of methane was produced at lower enzymes dosages for office paper and magazines, the decrease in methane production between the enzyme dosages was quite small for all the fractions.

Furthermore, cardboard and newspaper did not produce less biogas when less enzyme was used in the fermentation experiments.

While the methane production was quite similar within a fraction, it was significantly different between the fractions. Only the newspaper and magazine fractions had almost identical methane yields (Figure 10 and Figure 11). The bars on Figure 11 resemble those of dissolved COD (but not total COD) from Figure 2 where the effect of the enzyme dosage on dissolved and total COD was studied. To quantify the relationship between dissolved COD and methane produced, the expected methane yield per gram of a fraction was calculated and compared with the experimental values. The methane yield was calculated as follows:

$$V_{CH4} = 0,35 \times COD_{diss}[g/gTS]/TS_{frac} \qquad (2)$$

where $COD_{diss}$ is dissolved COD (in g/g TS) and $TSf_{rac}$ is the dry matter of a fraction.

By plotting the difference of the actual methane produced versus the calculated by using formula (2), one can see that several fractions yielded consistently more methane than expected from only dissolved COD while for several other fractions the difference was close 0% and there was only one experiment where much less methane than expected was produced (Figure 12). Most likely, the noticeable positive difference is due to a large contribution of suspended COD in the total methane yield. Indeed, all the experiments with a pronounced second peak in the methane production rate are among those with a large positive difference between the actual and calculated methane production. It is worth noting that all the cardboard fractions regardless of the conditions during the enzymatic hydrolysis produced more methane that expected. This suggests that the enzymes are unable to fully hydrolyse the cardboard fractions, which does not lead to a lower methane yield (assuming that the suspended cardboard particles can reach biogas bacteria). At the same time, suspended COD did not have a big impact on the methane production from most of the fractions and most of methane was presumably produced from dissolved COD.

The current study estimated the methane potential of cellulosic fractions. As is evident from Figure 13, the methane production was quite specific for each fraction giving the following average values ($\pm$ standard deviation): office paper 207 $\pm$ 29 NmL/g fraction, cardboard 143 $\pm$ 14 NmL/g fraction, newspaper 54 $\pm$ 31 NmL/g fraction, magazine 47 $\pm$ 31 NmL/g fraction (Table 2). The higher the grade of the fractions the higher the methane yield.

**Table 2. Average methane yield for the cellulosic fractions studied**

| Fraction | Average CH$_4$ NmL/g fraction | Stdev CH$_4$ NmL/g fraction |
|---|---|---|
| Office paper | 207 | 29 |
| Cardboard | 143 | 14 |
| Newspaper | 54 | 31 |
| Magazine | 47 | 31 |

Composition of the fractions:

- the used fractions were found to be representative, and the results obtained with these fractions are expected to be similar for any kind of office paper, cardboard, newspapers and magazines.

Fermenter experiments:

- the increase in size of the fractions and pre-soaking in warm water might have a small negative effect on the dissolved COD of the hydrolysis,
- reducing the enzyme dosage reduces the dissolved COD while the total COD was only mildly affected for all the tested fractions,
- acidified reject water did not have any negative effects of the performance of the enzymes,
- the addition of the cellulosic fractions to fermentation of model waste increases the dissolved COD,
- Enzymes can fully hydrolyse and dissolve only a part of cellulose while partially hydrolyse the rest of cellulose. This loosens the structure of the fractions and facilitates their complete breakdown which makes it possible to bring a large part of the fractions to AD even though they were not fully dissolved,

AMPTS experiments:

- pre-soaking of the fractions in warm water prior to enzymatic hydrolysis had almost no effect on the methane production and yield

- the enzyme dosage in the enzymatic fermentations had a small effect on the methane yield in the AMPTS experiments, however, at low enzyme dosages a significant amount of methane was produced from suspended but not dissolved COD
- Enzymes might have a limited capacity to fully hydrolyzing cellulose from cardboard because cardboard was the only fraction for which suspended COD had a large contribution in the methane yield regardless of conditions tested,

**Analysis of the contaminated cellulosic fraction obtained from MSW**

Source segregated, mixed contaminated cellulosic fraction has been sampled at transfer station and compositional analyses was performed on this sample. The sample contains different fractions, including contamination from source sorting - possibly plastic fractions added at household and some of the contamination is cross contamination from transfer station. The material has been stored outside in open bay, where atmospheric conditions like wind and rain were partly causing some contamination (soil, feather and hair).

The composition of the contaminated cellulosic material from waste is listed as fractions in Table 3 below:

**Table 3. Composition of contaminated cellulosic fraction from MSW**

|  | Weight (g) |
|---|---|
| **Paper (sum)** | 1758 |
| Newspaper | 3 |
| Receipts | 6 |
| Fotos/postcards | 17 |
| Birthday/Christmas/visiting cards | 103 |
| Magazines | 305 |
| Office paper | 1231 |
| Napkins/paper handkerchiefs | 93 |
| **Cardboard** | 1077 |
| **juice cartons** | 164 |
| **Window envelopes** | 99 |
| **Plastic soft** | 126 |
| **Plastic hard** | 14 |
| **Metal** | 20 |
| **Wood** | 42 |
| **Baq with used tampons** | 61 |
| **soil, hair and feather** | 14 |
| **SUM** | 3375 |

Moreover, the fractions of the contaminated cellulosic material from MSW are shown in Figure 14. The fractions in figure 14 is shown in the same order from left to right as listed in Table 3: Newspaper, receipts, fotos/postcards, Birthday/Christmas/visiting cards, magazines, office paper, napkins/paper handkerchiefs, cardboard, juice cartons, window envelopes, soft plastic, hard plastic, metal, wood, plastic bag with used tampons, and soil/hair/feather.

**References**

1. Irina Naroznova, Jacob Moller, and Charlotte Scheutz, Characterisation of the biochemical methane potential (BMP) of individual material fractions in Danish source-separated organic household waste, Waste Management, Volume 50, 2016, Pages 39-48.

2. Yuan et.al., Bioresource Technology, Volume 118, August 2012, Pages 281-288.

3. Guilford., et.al., Environ. Sci. Technol. 2019, 53, 21, 12677-12687.

4. Shahbaz, M., Ammar, M., Korai, R.M. et al., Impact of C/N ratios and organic loading rates of paper, cardboard and tissue wastes in batch and CSTR anaerobic digestion with food waste on their biogas production and digester stability. SN Appl. Sci. 2, 1436 (2020).

5. Gonzalez-Estrella, J., Asato, C.M., Stone, J.J. et al., A review of anaerobic digestion of paper and paper board waste. Rev Environ Sci Biotechnol 16, 569-590 (2017).

**Claims**

1. A method for processing cellulosic material, wherein the method comprises the steps of:

   (i) adding a stream of cellulosic material, such as paper, cardboard, pizza trays, carton, newspapers, magazines, office paper, diapers, pulp or paper sludge, to a bioreactor and optionally add liquid, wherein the cellulosic material has a dry matter content of cellulose of at least 20% by dry weight;
   (ii) further add an enzyme comprising one or more cellulase enzymes selected from the group comprising an endoglucanase, a cellobiohydrolase, and a beta-glucosidase; followed by
   (iii) enzymatic hydrolysis of the cellulosic material within the bioreactor at a temperature in the range of 35-75 °C and pH 4-7 for 2-48 hours resulting in liquefaction of the biodegradable organic matter and the cellulosic material to produce a bioliquid; optionally followed by
   (iv) separation of liquid and solid; optionally followed by
   (v) anaerobic fermentation of the bioliquid into a biogas.

2. The method according to claim 1 wherein the cellulosic material is contaminated with biodegradable, and/or inert and/or non-biodegradable material, such as food waste, biogernic material, plastics, sand, metal, non-biodegradable combustible material, material isolated from waste, such as municipal solid waste or sludge, such as pulper or paper sludge, sludge from wastewater treatment plant, preferably wherein the contaminated cellulosic material comprises between 20 and 99% by weight cellulosic material, preferably between 30 and 80% by weight cellulosic material, more preferably between 40 and 70% by weight cellulosic material of the treated stream.

3. The method of any of the preceding claims, wherein the cellulosic material comprises between 30 and 99% by dry weight cellulose, preferably between 30 and 80% by weight cellulose, more preferably between 35 and 60% by weight cellulose on a dry matter basis.

4. The method of any of the preceding claims, wherein the cellulosic material comprises between 0 and 40% by weight hemicellulose, preferably between 1 and 25% by weight hemicellulose, more preferably between 5 and 25% by weight hemicellulose on a dry matter basis and/or wherein the cellulosic material comprises between 0 and 30% by weight lignin, preferably between 0 and 25% by weight lignin, more preferably between 1 and 25% by weight, most preferably between 5 and 25% by weight lignin on a dry matter basis.

5. The method according to any of the proceeding claims, wherein the enzymes are added as recirculated bioliquid, process water, microorganism secreting enzyme, or as an enzyme composition.

6. The method of any of the preceding claims, wherein the cellobiohydrolase is one or more enzymes selected from the group comprising a cellobiohydrolase I, a cellobiohydrolase II, and a beta-glucosidase, and/or wherein the hemicellulase is one or more enzymes selected from a xylanase, an acetylxylan esterase, a feruloyl esterase, an arabinofuranosidase, a xylosidase, and a glucuronidase.

7. The method of any of the preceding claims, wherein the enzymatic hydrolysis is performed at a temperature in the range of 40-60 degrees C, more preferably 45-55 degrees C, most preferably 50 degrees C.

8. The method of any of the preceding claims, wherein the enzymatic hydrolysis is performed at a pH in the range of 4-6, more preferably 4.5-5.5, most preferably 5, and/or wherein the enzymatic hydrolysis is performed for a period of wherein the enzymatic hydrolysis is performed for a period of 10-48 hours, preferably 15-30 hours, more preferably 20-30 hours, most preferably 24 hours.

9. The method of any of the preceding claims, wherein the cellulosic material is subjected to fermentation concomitantly with or after the enzymatic hydrolysis to further liquify the cellulosic material.

10. The method of any of the preceding claims, wherein the anaerobic fermentation is conducted at a temperature in the range of 30-75 degrees C, preferably 40-60 degrees C, more preferably 45-55 degrees C, most preferably 50 degrees C.

11. The method of any of the preceding claims, wherein the anaerobic fermentation is performed for a period of 10-48 hours, preferably 15-30 hours, more preferably 20-30 hours, most preferably 24 hours.

12. The method of any of the preceding claims, wherein the methane potential of the liquefied cellulosic material is 25-250 L/kg dry cellulosic material, preferably 50-250 L/kg cellulosic material, more preferably 100-250 L/kg dry cellulosic material and most preferably 150-250 L/kg dry cellulosic material.

13. The method of any of the preceding claims, wherein

the anaerobic fermentation in step (iv) is conducted by transferring the bioliquid into an anaerobic digestion system; followed by
conducting anaerobic fermentation of the bioliquid to produce biogas, and wherein the anaerobic fermentation is conducted at a pH between 6.0 and 9.0, preferably between 6.5 and 8.5, most preferred 8.0.

14. Use of a cellulosic material for producing a bioliquid suitable as a biogas substrate comprising the steps of:

(i) adding a stream of cellulosic material, such as paper, cardboard, pizza trays, carton, newspapers, magazines, office paper, diapers, pulp or paper sludge, to a bioreactor and optionally add liquid, wherein the cellulosic material has a dry matter content of cellulose of at least 20% by dry weight;
(ii) further add an enzyme comprising one or more cellulase enzymes selected from the group comprising an endoglucanase, a cellobiohydrolase, and a beta-glucosidase and one or more enzymes selected from the list comprising a protease, a lipase, a beta-glucanase, a pectate lyase, a hemicellulase, a mannanase, and an amylase; followed by
(iii) enzymatic hydrolysis of the cellulosic material within the bioreactor at a temperature in the range of 35-75 °C and pH 4-7 for 2-48 hours resulting in liquefaction of the biodegradable organic matter and the cellulosic material to produce a bioliquid; optionally followed by
(iv) separation of liquid and solid; optionally followed by
(v) anaerobic fermentation of the bioliquid into a biogas.

15. A biogas substrate obtained from a cellulosic material obtained by the process of:

(i) adding a stream of cellulosic material, such as paper, cardboard, pizza trays, carton, newspapers, magazines, office paper, diapers, pulp or paper sludge, to a bioreactor and optionally add liquid, wherein the cellulosic material has a dry matter content of cellulose of at least 20% by dry weight;
(ii) further add an enzyme comprising one or more cellulase enzymes selected from the group comprising an endoglucanase, a cellobiohydrolase, and a beta-glucosidase and one or more enzymes selected from the list comprising a protease, a lipase, a beta-glucanase, a pectate lyase, a hemicellulase, a mannanase, and an amylase; followed by
(iii) enzymatic hydrolysis of the cellulosic material within the bioreactor at a temperature in the range of 35-75 °C and pH 4-7 for 2-48 hours resulting in liquefaction of the biodegradable organic matter and the cellulosic material to produce a bioliquid; optionally followed by
(iv) separation of liquid and solid; optionally followed by
(v) anaerobic fermentation of the bioliquid into a biogas.

Fig. 1

Fig. 2

Fig. 3

Fig. 4

Fig. 5

Relative COD (after 24 h) at different enzyme dosages

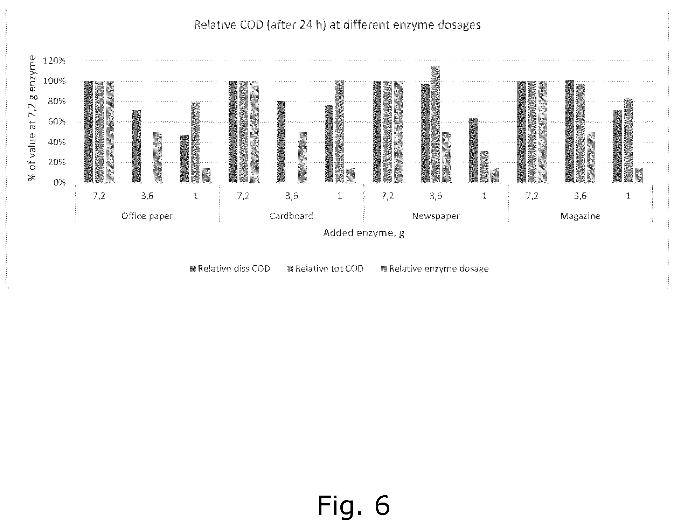

Fig. 6

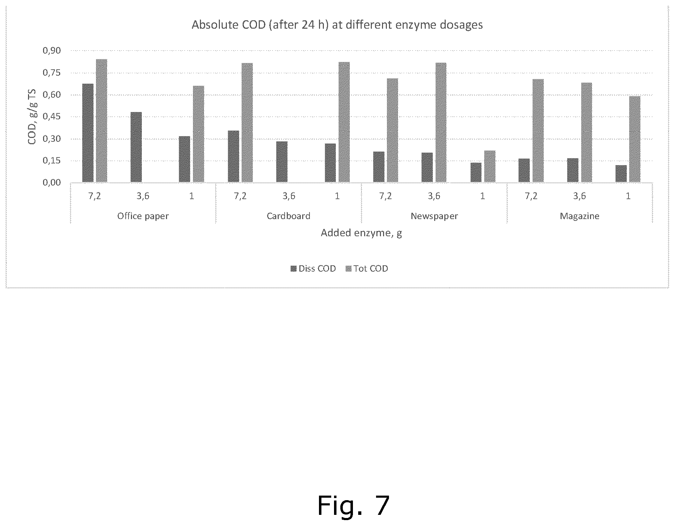

Fig. 7

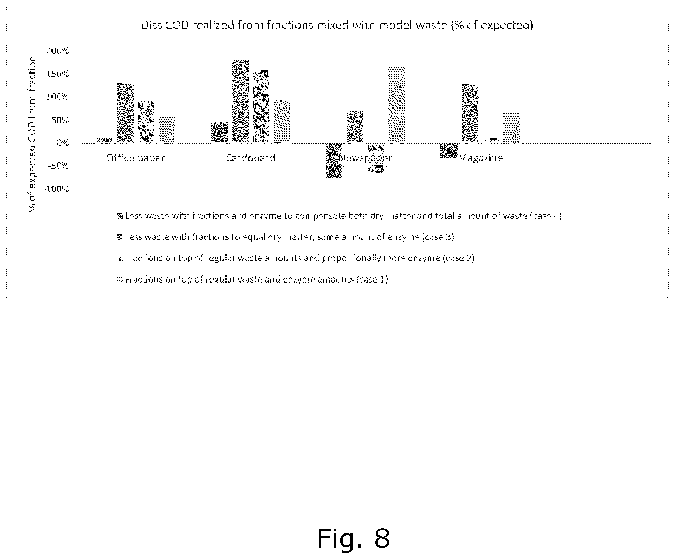

Fig. 8

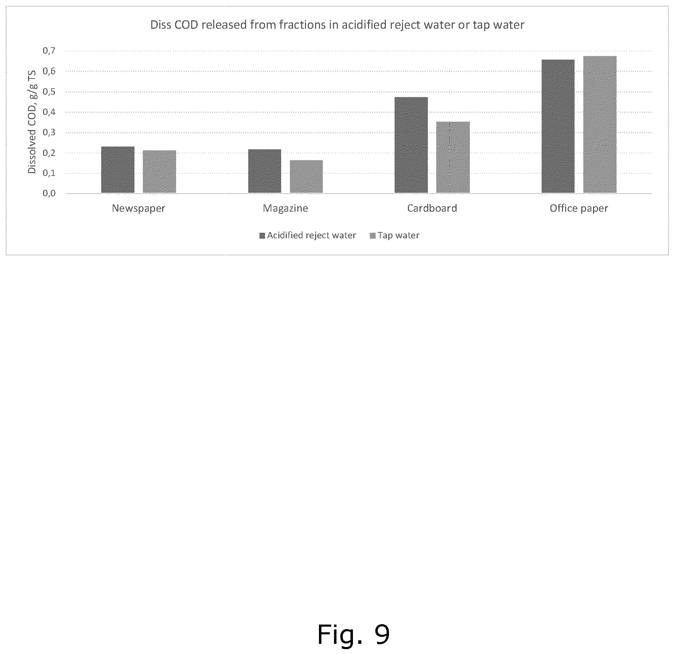

Fig. 9

Fig. 10

Fig. 11

Fig. 12

Fig. 13

Fig. 14

Europäisches
Patentamt

European
Patent Office

Office européen
des brevets

# EUROPEAN SEARCH REPORT

**Application Number**

EP 24 17 9299

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/091231 A1 (NAT UNIV IRELAND [IE]; TUOHY MARIA GERARDINE [IE] ET AL.) 16 August 2007 (2007-08-16) * page 48 - page 50; example; table 18 * * table 16 * * example 7(b) * * abstract; claims 17, 23, 78, 79 * ----- | 1-8, 10-15 | INV. C12P5/02 ADD. C12P19/02 C12P19/14 |
| X | WO 2016/209183 A1 (EPISOME BIYOTEKNOLOJIK ÜRÜNLER SANAYI VE TICARET ANONIM SIRKETI [TR]) 29 December 2016 (2016-12-29) * page 1, paragraph 1 * * claims * ----- | 1-10,12, 14,15 | |
| X | WO 2011/092136 A1 (NOVOZYMES AS [DK]; OLSEN HANS SEJR [DK]) 4 August 2011 (2011-08-04) * claims * * page 4, paragraph 2 * ----- | 1-7,12, 14,15 | |

**TECHNICAL FIELDS SEARCHED (IPC)**

C12P

The present search report has been drawn up for all claims

| Place of search | Date of completion of the search | Examiner |
|---|---|---|
| Berlin | 6 December 2024 | Schröder, Gunnar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 9299

This annex lists the patent family members relating to the patent documents cited in the above-mentioned European search report.
The members are as contained in the European Patent Office EDP file on
The European Patent Office is in no way liable for these particulars which are merely given for the purpose of information.

06-12-2024

| Patent document cited in search report | | | Publication date | Patent family member(s) | | Publication date |
|---|---|---|---|---|---|---|
| WO 2007091231 | A1 | 16-08-2007 | | AU | 2007213401 A1 | 16-08-2007 |
| | | | | BR | PI0707893 A2 | 10-05-2011 |
| | | | | CA | 2642133 A1 | 16-08-2007 |
| | | | | CN | 101460614 A | 17-06-2009 |
| | | | | EP | 1989300 A1 | 12-11-2008 |
| | | | | EP | 2363459 A1 | 07-09-2011 |
| | | | | JP | 5329237 B2 | 30-10-2013 |
| | | | | JP | 2009525747 A | 16-07-2009 |
| | | | | PL | 1989300 T3 | 28-02-2013 |
| | | | | US | 2010028485 A1 | 04-02-2010 |
| | | | | WO | 2007091231 A1 | 16-08-2007 |
| WO 2016209183 | A1 | 29-12-2016 | | BR | 112017027665 A2 | 28-08-2018 |
| | | | | CA | 2986565 A1 | 29-12-2016 |
| | | | | CN | 107709570 A | 16-02-2018 |
| | | | | DK | 3314003 T3 | 15-04-2019 |
| | | | | EA | 201792457 A1 | 30-03-2018 |
| | | | | EP | 3314003 A1 | 02-05-2018 |
| | | | | ES | 2720064 T3 | 17-07-2019 |
| | | | | JP | 6464443 B2 | 06-02-2019 |
| | | | | JP | 2018522534 A | 16-08-2018 |
| | | | | KR | 20170141244 A | 22-12-2017 |
| | | | | US | 2018155750 A1 | 07-06-2018 |
| | | | | WO | 2016209183 A1 | 29-12-2016 |
| WO 2011092136 | A1 | 04-08-2011 | | BR | 112012018422 A2 | 15-09-2015 |
| | | | | CA | 2788548 A1 | 04-08-2011 |
| | | | | CN | 102884197 A | 16-01-2013 |
| | | | | EP | 2529022 A1 | 05-12-2012 |
| | | | | US | 2013040354 A1 | 14-02-2013 |
| | | | | WO | 2011092136 A1 | 04-08-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013185778 A
- WO 2013185777 A
- WO 2014198274 A

**Non-patent literature cited in the description**

- **GUILFORD**. *Environ. Sci. Technol.*, 2019, 53
- **SHAHBAZ**. *Appl. Sci.*, 2020, vol. 2, 1436
- **NAROZNOVA**. *Waste Management*, 2016, vol. 50
- **IRINA NAROZNOVA** ; **JACOB MOLLER** ; **CHARLOTTE SCHEUTZ**. Characterisation of the biochemical methane potential (BMP) of individual material fractions in Danish source-separated organic household waste. *Waste Management*, 2016, vol. 50, 39-48
- **YUAN**. *Bioresource Technology*, August 2012, vol. 118, 281-288
- **GUILFORD.** *Environ. Sci. Technol.*, 2019, vol. 53 (21), 12677-12687
- **SHAHBAZ, M.** ; **AMMAR, M.** ; **KORAI, R.M. et al.** Impact of C/N ratios and organic loading rates of paper, cardboard and tissue wastes in batch and CSTR anaerobic digestion with food waste on their biogas production and digester stability. *SN Appl. Sci.*, 2020, vol. 2, 1436
- **GONZALEZ-ESTRELLA, J.** ; **ASATO, C.M.** ; **STONE, J.J. et al.** A review of anaerobic digestion of paper and paper board waste.. *Rev Environ Sci Biotechnol*, 2017, vol. 16, 569-590